# EUROPEAN PATENT APPLICATION

(11) **EP 4 660 293 A1**
(43) Date of publication of application: **10.12.2025**
(21) Application number: 24382613.8
(22) Date of filing: 06.06.2024
(51) Int. Cl.: C12N 1/20, A01N 63/22, C05F 11/08, C12R 1/07

(54) **BACILLUS VELEZENSIS STRAIN AND USES THEREOF**

(71) Applicant: VitaNtech Biotechnology SL, 18003 Granada (ES)
(72) Inventor: Barros Rodríguez, Adoración, Granada (ES); Manzanera Ruiz, Maximino Enrique, Granada (ES)
(74) Representative: Pons IP

(57) **Abstract**

The present invention relates to a new *Bacillus velezensis* strain with deposit number CECT 30599 as well as uses thereof in agriculture. Specifically, the present invention relates to the use of the *8. velezensis* strain CECT 30599 to improve the resistance of a plant to drought, promote germination in a plant, promote the growth of a plant, the control of pathogenic microorganisms in a plant, reduce the water supply to a plant, promote flowering in a plant, and/or increase the population of plant growth-promoting rhizobacteria in the soil where a plant is grown.

## Description

The present invention relates to a new *Bacillus velezensis* strain and uses thereof in the field of agriculture. Therefore, the invention could be comprised in the field of biotechnology.

### STATE OF THE ART

Throughout the last century, the human population has experienced a significant increase, and this translates into an increase in the need to provide food for such population. Because of that, the field of agriculture has experienced an increase in production to meet the nutritional needs of the growing population, which has led farmers to use intensive agriculture techniques and improve production to meet the growing demand.

The resources used to improve production include the use of chemical compounds which allow plant growth or protection against pathogenic organisms (agrochemicals) to be increased. However, the use of agrochemicals entails negative effects that can affect the environment in which they are used as these are compounds that persist in the environment, and furthermore, they can be toxic not only to other plant species or other organisms (such as soil microbiota in the environment), but they can also be toxic to consumers of foods from crops on which they are applied. Therefore, when proposing new crop improvement strategies, it is necessary to take these adverse effects into account (Carvalho, F. P. (2006). Environmental science & policy, 9(7-8), 685-692).

Another important factor when proposing strategies to increase agricultural production is that climate change is becoming more noticeable. One of the main causes of climate change is the increase in greenhouse gas emissions, which is causing an increase in temperatures globally. This increase in global temperature directly affects agricultural production, as more and more regions suffer from recurring periods of drought, causing an increase in the respiration rate of crops and evapotranspiration, increased pest infestation, a change in weed flora, reduction of crop duration, and also affects the microbial population and its enzymatic activities in the soil (Malhi, G. S., Kaur, M., & Kaushik, P. (2021). Sustainability, 13(3), 1318).

In regions with water scarcity, like many hot areas, agriculture can put great pressure on available water resources. This leads to the need to overuse aquatic resources, like rivers, lakes and aquifers, for irrigation of crops. Excessive water extraction can deplete natural resources, reduce river flows, reduce groundwater levels and cause soil salinisation, which negatively affects both water and land ecosystems.

Due to the above, it is becoming increasingly necessary to implement sustainable agriculture strategies which are more environment friendly and adapted to the situation of climate change, at the same time that they allow the demand of a continuously growing population to be met (Velten, S., Leventon, J., Jager, N., & Newig, J. (2015). Sustainability, 7(6), 7833-7865).

In this regard, in recent years, the use of microorganism-based strategies has been progressively adopted to improve crop yields, thus replacing the use of agrochemicals. Furthermore, the use of microorganism-based strategies increases plant resistance to environmental stresses (Saints, M. S., Nogueira, M. A., & Hungria, M. (2019). Amb Express, 9(1), 205).

Therefore, there is a need to provide new microbiological strategies capable of improving the performance of crop plants, improving their growth rate, resistance to pathogens and resistance to adverse environmental conditions such as drought, at the same time that they form a more sustainable and environmentally friendly alternative, than agrochemical-based strategies.

### DESCRIPTION OF THE INVENTION

The present inventors have discovered a novel *Bacillus velezensis* strain with deposit number CECT 30599, which has a multitude of beneficial properties for plants.

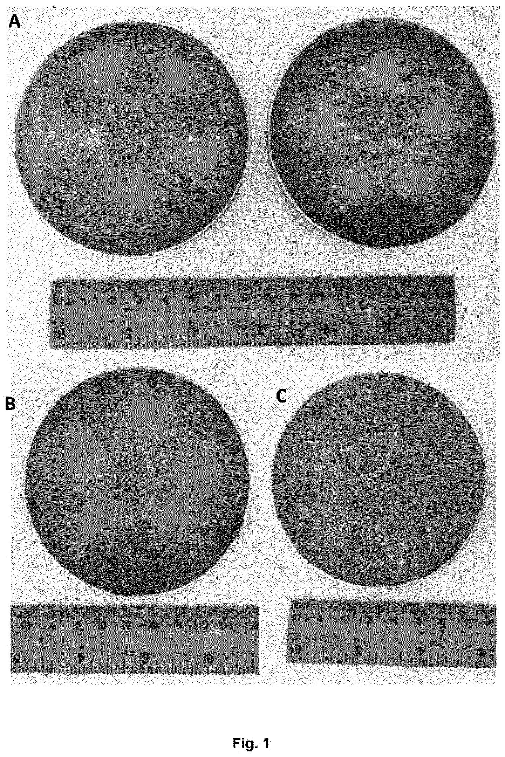

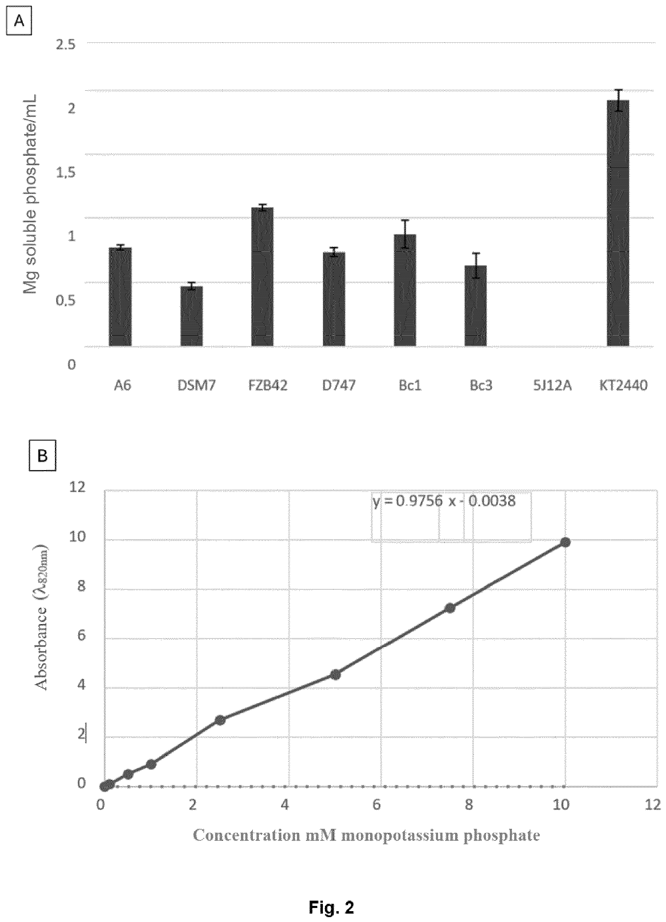

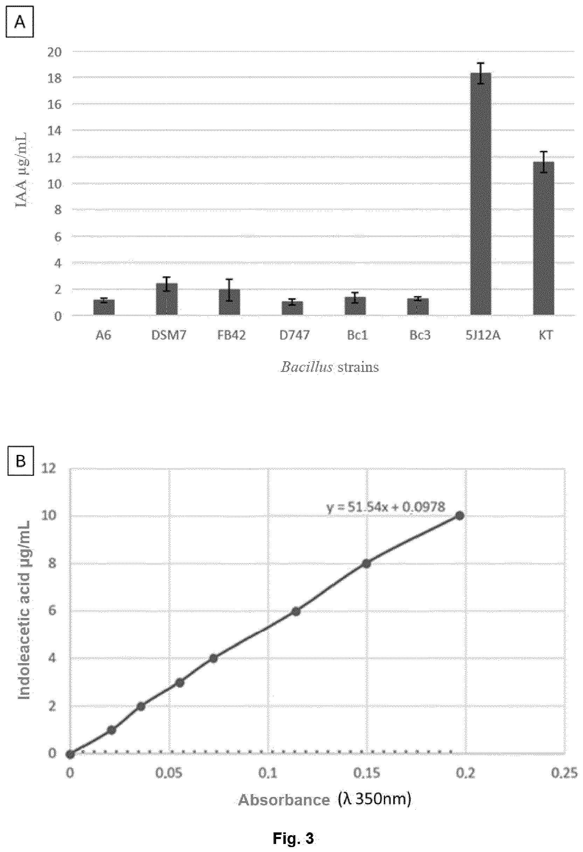

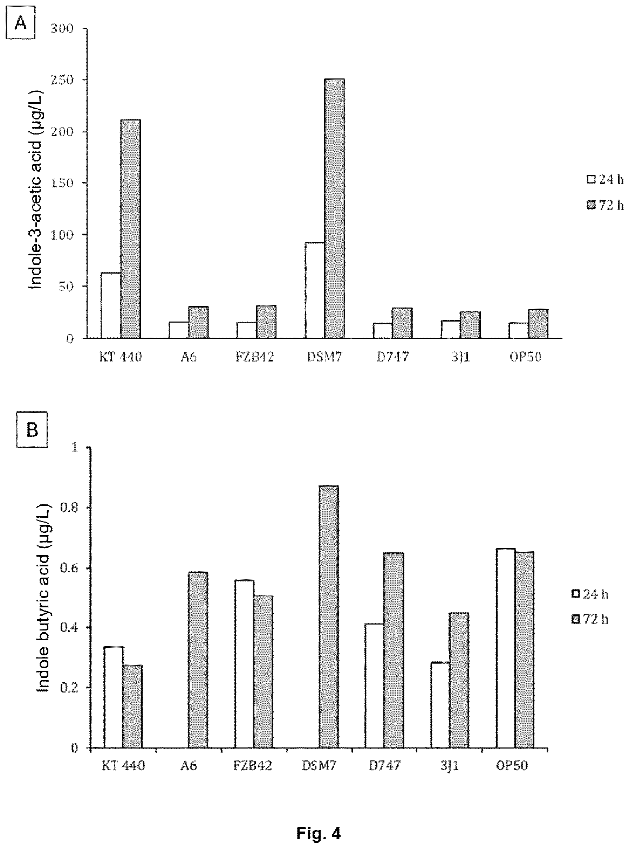

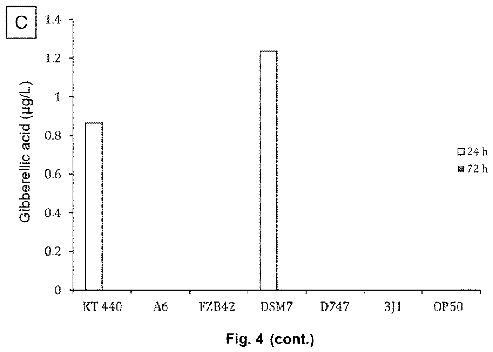

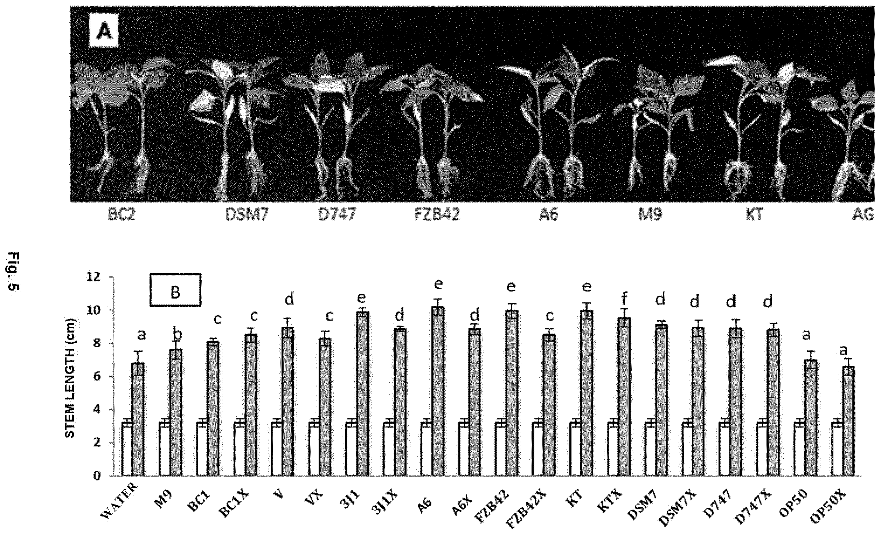

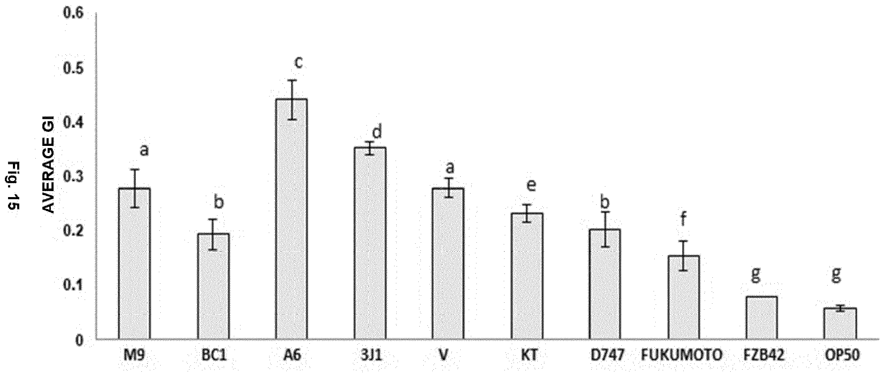

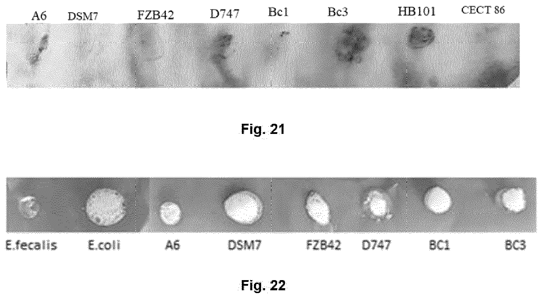

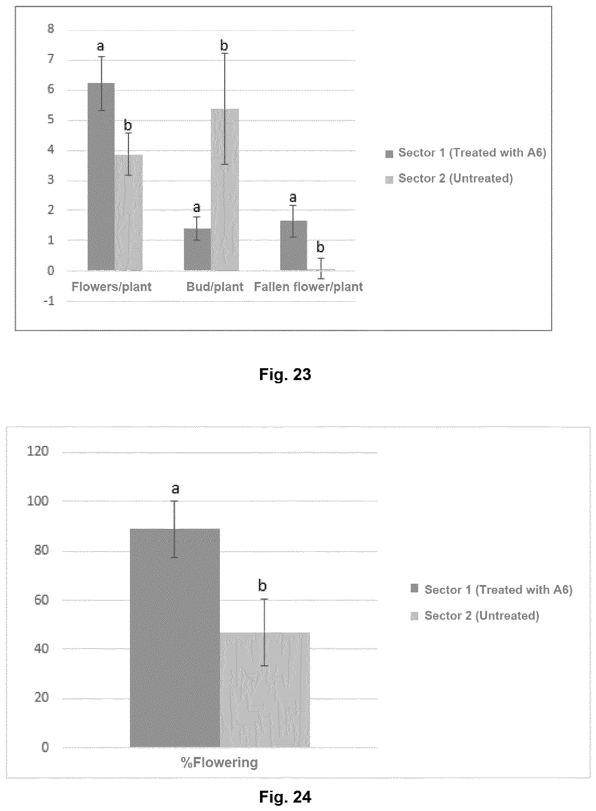

The strain of the invention has growth-promoting activity for the plants to which it is administered (Figures 5 to 8 and Figures 10 to 13). Furthermore, the strain of the invention presents activities related to promoting the growth and development of a plant, such as: phosphate solubilisation capacity, which improves the absorption thereof (Figure 1 and Figure 2), production of phytohormones (Figure 3 and 4), urease activity, which is essential for the urea metabolism and nitrogen absorption, cytochrome oxidase activity (Figure 21) and catalase activity which have an antioxidant effect by eliminating reactive species (Figure 22). Furthermore, the strain of the invention is also capable of significantly increasing the germination rate, which translates into a greater number of plants that develop from the seed stage to the adult stage (Figure 15). It is also capable of promoting flowering in a plant to which it is administered (Fig. 23 and Fig. 24).

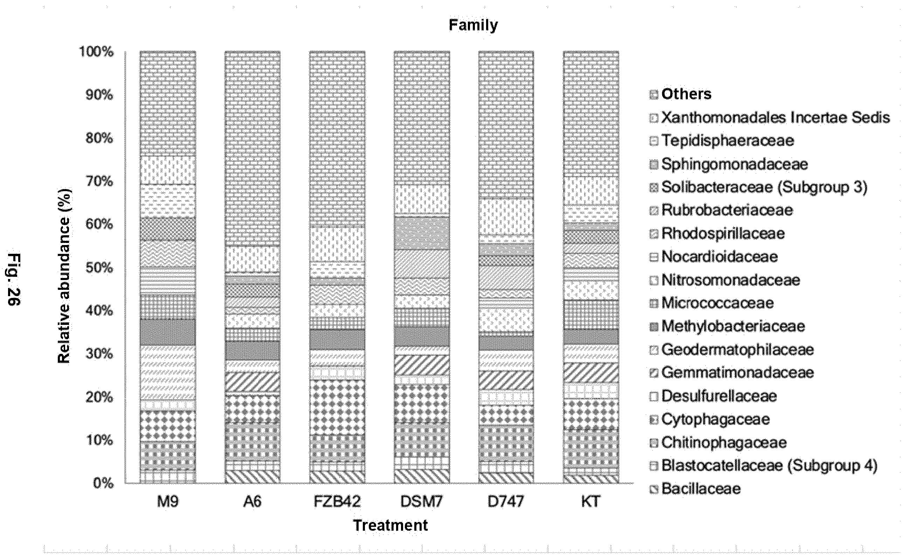

Surprisingly, the strain of the invention promotes the improvement of the biodiversity of plant growth-promoting rhizobacteria (PGPR) in the soil (Figure 26). Soil nutrition plays a fundamental role in the growth and development of plants. In this sense, soil microorganisms play a crucial role in contributing to nutrient availability through their PGPR activities. The introduction of the strain of the invention has proven to be an effective strategy to improve the health and productivity of the crop, by increasing the proportion of PGPR bacteria in the soil, which is something that had not been identified to date. This potential ability of a microorganism to activate and enhance the activities of other PGPRs is of great importance when it comes to promoting activities in the soil.

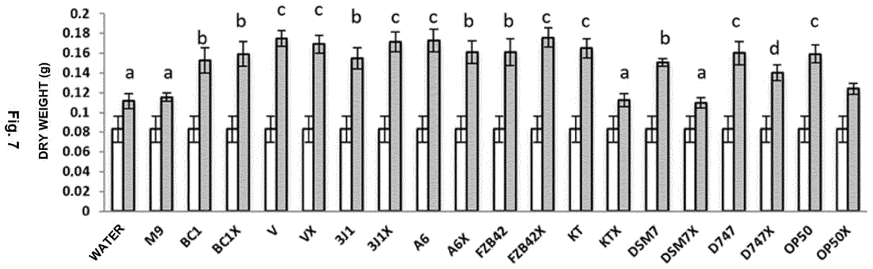

Advantageously, the strain of the invention also improves the tolerance of plants to drought, giving rise to better fresh weight values, dry weight, relative water content/water holding capacity (Figure 7 to 9, Figure 12 to 14 and Figure 20). In the presence of the strain of the invention, a reduction in the amount of water that must be administered to a plant for its growth is also observed. Therefore, it is presented as an advantage that allows saving water resources used for irrigation in agriculture.

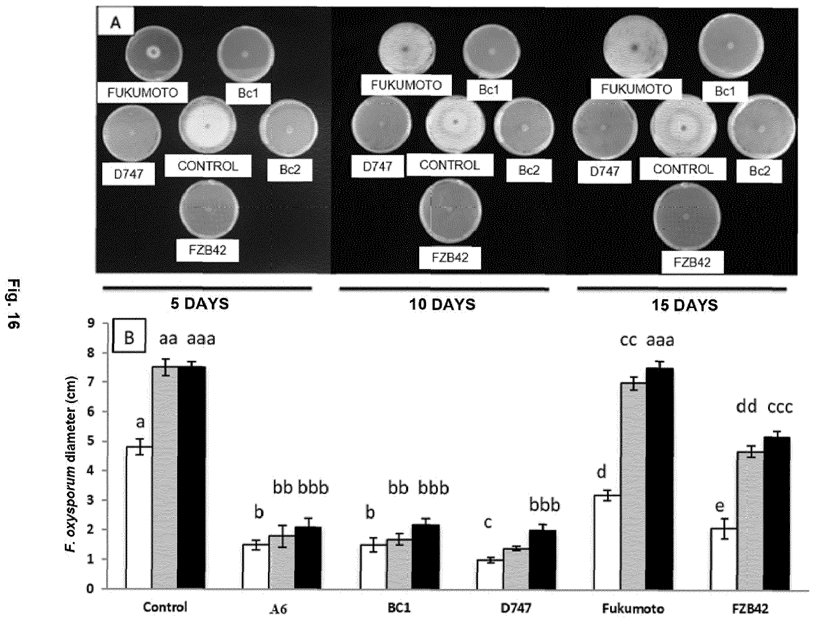

Furthermore, the strain of the invention, also produces secondary metabolites related to plant defence (table 3) and is capable of reducing the incidence of phytopathogenic microorganisms, thus exerting a phytoprotective effect on plants to which it is administered (Figures 16 to 19 and Figure 25).

Therefore, in light of the aforementioned, the new strain provided by the present inventors presents a series of characteristics/activities that are of great interest in the field of agriculture, entailing a new, more sustainable and environmentally friendly alternative to strategies based on agrochemicals.

Therefore, in a first aspect, the present invention relates to a *Bacillus velezensis* strain with the deposit number CECT 30599 or a mutant thereof, hereinafter "strain or mutant of the invention" respectively.

The *Bacillus velezensis* strain with deposit number CECT 30599 was deposited in the Spanish Type Culture Collection (with address Parc Cientific Universitat de Valencia, calle del Catedrático Agustin Escardino, 9, 46980 Paterna (Valencia) Spain) under the Budapest Treaty for patent purposes, on 30 March 2022. The *B. velezensis* strain with deposit number CECT 30599 was isolated from a sample of soil from dry land taken in the town of Las Gabias, province of Granada, with geographical coordinates 37°10'55" N, 3°41'20" W.

In particular, the members of the species *B. velezensis,* are safe as they are considered GRAS by the FDA in the United States and QPS by the EFSA in the European Union ("*Qualified presumption of safety*"*,* which is a term similar to the well-known GRAS, "*generally recognized as safe").* Accordingly, the strain of the invention is suitable for use in agriculture.

The term "mutant" refers to a strain that comprises at least one variation or mutation in the genome thereof with respect to the genome of the "wild-type" strain from which it is derived or from which it is obtained. The variation in the genome can be a point mutation, an insertion, a deletion, or combinations thereof, in which the variation or variations in the genome of the mutant strain do not affect cell viability.

In the present invention, a mutant of the strain of the invention refers to a mutant obtained using the *B. velezensis* strain CECT 30599 as starting material.

A "mutant" of *B. velezensis* CECT 30599 is also understood according to the invention as a "variant" of *B. velezensis* CECT 30599.

A "mutant" of *B. velezensis* CECT 30599 or mutant of the invention, retains the characteristics of the strain of the invention. Therefore, in a preferred embodiment, the mutant of the invention maintains the following capabilities or characteristics:
- improve the resistance of a plant to drought,
- promote germination in a plant,
- promote the growth of a plant,
- the control of pathogenic microorganisms in a plant,
- reduce the water supply to a plant,
- promote flowering in a plant, and/or
- increase the proportion of plant growth-promoting rhizobacteria in the soil where a plant is grown.

Those skilled in the art will understand that mutants that retain the relevant features and advantages described herein can be obtained routinely, for example, by means of spontaneous mutagenesis or directed mutation, using the strain of the invention as starting material. Methods for obtaining mutants of a specific bacterial strain are known in the state of the art. An example can be found in Sambrook, J. and Russell, D.W. "Molecular Cloning: A Laboratory Manual", Chapter 13, "Mutagenesis", Cold Spring Harbor, 3rd Ed, 2001. In the present invention the term "strain of the invention" refers not only to the *B. velezensis* strain CECT 30599 but also to mutants thereof.

In another preferred embodiment, the mutant of the invention has a genome that has a sequency identity of at least 99.5% with the genome of the *B. velezensis strain* CECT 30599, preferably has a sequence identity of 99.6%, 99.7%, 99.8% or 99.9% with the genome of the *B. velezensis strain* CECT 30599.

In the present invention, "identity" or "sequence identity" is understood as the degree of similarity between two nucleotide or amino acid sequences obtained by aligning the two sequences. Depending on the number of common residues between the aligned sequences, a degree of identity expressed as a percentage is obtained. The degree of identity between two nucleotide/amino acid sequences can be determined by conventional methods, for example, using standard sequence alignment algorithms known in the state of the art, such as BLAST [Altschul S.F. et al. Basic local alignment search tool. J Mol Biol. 1990 Oct 5; 215(3):403-10]. The BLAST programs, for example, BLASTN, BLASTX, and TBLASTX, BLASTP and TBLASTN, are in the public domain on the website of *The National Center for Biotechnology Information* (NCBI).

The strain of the invention is referred to as "*B*. *velezensis* with deposit number CECT30599", "*B*. *velezensis* CECT30599, "A6 strain" or "*B*. *velezensis A6",* interchangeably, throughout the description.

As is known to the person skilled in the art, the capability of improving the resistance of a plant to drought, promoting germination in a plant, promoting the growth of a plant or controlling pathogenic microorganisms in a plant, both strain of the invention and a mutant strain thereof, can be determined through routine practice assays. The improvement in resistance to drought can be established by evaluating the water retention capacity of a plant treated with a bacterial strain or a mutant thereof. The promotion of germination in a plant can be evaluated by carrying out an assay in which the germination index of a plant treated with a bacterial strain or a mutant thereof is established. The promotion of plant growth can be evaluated by carrying out an assay in which the absolute growth or growth rate of a plant treated with a bacterial strain or a mutant thereof is determined. The assays mentioned in this paragraph may also comprise a comparison with a plant or group of plants not treated with the bacterial or mutant strain thereof that is assayed. The control of pathogenic microorganisms in a plant can be evaluated by determining the microorganism inhibitory capacity of a bacterial strain or a mutant thereof. The reduction in water supply can be evaluated by determining the amount of water provided as irrigation (in a given period of time) to a plant to which a bacterial strain or a mutant is administered, compared to a plant to which said strain or mutant has not been administered. The promotion of flowering can be determined by evaluating the number of flowers on a plant or the number of plants that have flowers in a population to which a bacterial strain or a mutant thereof has been administered. The increase in plant growth-promoting rhizobacteria can be evaluated by determining the proportion of such rhizobacteria in a total population of microbiota in a sample of loose where a plant grows to which a bacterial strain or a mutant of the same assay is administered. Likewise, the determinations in the mentioned paragraph can be carried out in assays in which a spore of a bacterial strain or the inactivated form thereof is administered. Preferably, determining the capabilities of the strain of the invention, a mutant, spore or inactivated strain of the strain of the invention is carried out as in the examples of the present invention.

In another aspect, the present invention relates to a cell culture of the strain or mutant of the invention, hereinafter, "the culture of the invention".

In the present invention, the term "cell culture" refers to a cell or group of cells (for example, the strain or mutant of the invention) that are comprised in a substrate, preferably in which they have expanded or remain viable, and in which they are comprised. The substrate of the cell culture, also referred to as "culture" or "culture medium", contains all the minimum nutritional elements that allow the growth and/or survival of the bacteria that are present therein. The culture medium can be liquid or solid, therefore, the cell culture of the invention can be solid or liquid. Examples of culture media include, but are not limited to, Luria-Bertani (LB), Tripto-Casein Soy Broth (TSB), Nutrient Broth (NB) or M9 minimal medium.

The strain or mutant of the invention preferably refers to a live or viable cell, where a living or viable cell refers to a cell capable of dividing and/or carrying out its metabolic activity.

However, the present inventors have also found that inactivated cells of the strain of the invention maintain the activities related to the strain of the invention in its live or viable form.

Therefore, in another aspect the present invention relates to an inactivated cell of the strain of the invention or the mutant of the invention, wherein said inactivated cell is hereinafter referred to as "the inactivated cell of the invention".

In the present invention, the term "inactivated cell" refers to a cell that lacks the ability to divide and/or metabolic capacity. An inactivated cell can preserve its total structure, partially or not preserving its cellular structure, preferably the inactivated cell preserves its cellular structure. Methods for inactivating bacteria are known to a person skilled in the art, some examples being, but not limited to, the use of chemical agents (that is, glutaraldehyde, formalin or paraformaldehyde) and the application of heat, high pressure or UV irradiation.

In a preferred embodiment, the inactivated cell of the invention is obtained by applying heat, preferably by autoclave, more preferably by autoclave heat application at 121 °C, for 30 minutes.

In a preferred embodiment, the inactivated cell of the invention maintains the following capabilities or characteristics:
- improve the resistance of a plant to drought,
- promote germination in a plant,
- promote the growth of a plant,
- the control of pathogenic microorganisms in a plant,
- reduce the water supply to a plant,
- promote flowering in a plant, and/or
- increase the proportion of plant growth-promoting rhizobacteria in the soil where a plant is grown.

The strain of the invention belongs to the genus *Bacillus,* characterised in that bacteria belonging to said genus have the ability to produce spores.

Therefore, in another aspect the present invention, it relates to a spore of the strain of the invention or the mutant of the invention, hereinafter, "spore of the invention". The term "spore" has the normal meaning widely known to those skilled in the art, preferably it refers to a latent, protected and non-reproductive state of a bacteria, which can potentially give rise to a viable cell.

In another aspect, the present invention relates to a composition, hereinafter "composition of the invention" comprising one or more agriculturally acceptable compounds and:
i) The strain of the invention,
ii) The mutant of the invention,
iii) The culture of the invention,
iv) The inactivated cell of the invention, or
v) The spore of the invention.

In the present invention, the term "agriculturally acceptable compound" refers to a compound suitable for use in agriculture, where said compound does not interfere with the activity of the active compound(s) of said composition (that is, the strain, mutant or culture of the invention) and, also, the agriculturally acceptable compounds are not toxic to humans or the plant to which they are administered. Agriculturally acceptable compounds include, but are not limited to, plant strengtheners, nutrients, wetting agents, compounds that improve adhesion, buffer compounds, stabilisers, antioxidants, osmotic protectors or solar protectors.

Therefore, in another preferred embodiment of the composition of the invention, the agriculturally acceptable compound is selected from the list consisting of plant strengtheners, nutrients, wetting agents, compounds that improve adhesion, buffer compounds, stabilisers, antioxidants, osmotic protectors and solar protectors.

Examples of agriculturally acceptable compounds include, but are not limited to, alkyl sulphuric esters, alkyl aryl sulphonates, alkyl aryl ethers, alcohol ethoxylates, ethoxylated fatty acids, polyethylene glycol ethers, sugars, acids, potassium salts of fatty acids, sodium salts of fatty acids, carrageenan, carboxymethylcellulose, xanthan gum, lignosulfonic acid, calcium lignosulphonates, magnesium and zinc, silicon oxide, bentonites, glycerin, magnesium oxides, calcium, aluminium and iron, among others.

The composition of the invention may be formulated in any form suitable for use in agriculture.

Therefore, in another preferred embodiment, the composition of the invention is formulated as a solid formulation, liquid formulation, dense formulation (which refers to a liquid formulation with additives to increase its viscosity and density), granules, powder, wettable powder, suspension concentrate or emulsifiable concentrate.

In another preferred embodiment, the composition of the invention is a phytosanitary composition and/or fertiliser. The term "phytosanitary composition" refers to all substances intended for crop protection and/or biocontrol of pests and diseases. The term "fertiliser composition" refers to a composition that provides nutrients for the improvement of soil characteristics for greater plant development.

According to the results obtained by the present inventors, the strain of the invention has beneficial effects when applied to a plant. Therefore, the present invention also relates to a method for improving characteristics in a plant related to the growth and development of the plant, or with the capacity to resist environmental stresses.

Therefore, in another aspect, the present invention relates to a method, hereinafter, "improvement method of the invention", for:
- improving the resistance of a plant to drought,
- promoting the germination in a plant,
- promoting the growth of a plant,
- the control of pathogenic microorganisms in a plant,
- reducing the water supply to a plant,
- promoting the flowering in a plant, and/or
- increasing the proportion of plant growth-promoting rhizobacteria in the soil where a plant is cultivated,
wherein said method comprises applying to the plant, to a part thereof or the surroundings thereof, an effective amount of:
i) the strain of the invention,
ii) the mutant of the invention,
iii) the culture of the invention,
iv) the inactivated cell of the invention,
v) the spore of the invention, or
vi) the composition of the invention.

"Drought" refers to stress conditions in which the plant suffers from a lack of water, mainly due to an increase in ambient temperature, a decrease in associated precipitation and/or water evaporation. Therefore, the present invention makes it possible to improve resistance to stress situations of this type in an environmentally friendly manner. In the present invention "improving the resistance of a plant to drought" refers to increasing the capacity of a plant to retain water in its tissues when such plant is exposed to stress conditions due to lack of water. Such stress conditions due to lack of water can be prolonged over time, the present invention thereby allowing the plant to maintain its physiological functions and grow/develop under conditions of stress due to lack of water. The improvement in drought resistance is related to better values related to fresh weight, dry weight, relative water content/water retention capacity of the plant. The strain of the invention therefore makes it possible to improve the resistance of a plant against drought compared to a plant that has not been treated or brought into contact with said strain of the invention, in which case the water retention capacity can be at least 1.1 times, 1.2 times, 1.5 times, 1.7 times, 2 times, 2.5 times, 3 times, 4 times or 5 times higher compared to an untreated plant.

In relation to the foregoing, the strain of the invention allows better management and optimisation of water resources in agriculture since it allows the amount of water necessary for irrigation to be reduced. Therefore, the strain of the invention allows "the reduction of the water supply to a plant", which refers to the fact that the strain of the invention allows the reduction of the amount of water that needs to be supplied, through the irrigation process, to a plant for the development thereof. Therefore, the strain of the invention allows water for agriculture to be saved, preserving such resource in a more efficient way, especially in areas where periods of drought are frequent. The reduction in the amount of water for irrigation can be 10%, 20%, 30%, 40% or 50% with respect to the irrigation water used for an untreated plant (that is, to which the strain of the invention has not been applied).

Furthermore, the strain of the invention promotes germination which comprises the beginning of the development of the plant from its seed form. In particular, the present inventors observed that the strain of the invention is capable of increasing the germination rate, which corresponds to the number of plants that germinate in a population of plants to which the strain of the invention has been applied. The increase in germination rate can be at least 1.1 times, 1.2 times, 1.5 times, 1.7 times, 2 times, 2.5 times, 3 times, 4 times, 5 times, 10 times, 25 times, 50 times, 75 times or 100 times higher compared to a population of plants to which the strain of the invention has been applied.

In a preferred embodiment of the improvement method of the invention, promoting germination is a plant in the seed state, therefore, i) the strain of the invention, ii) the mutant of the invention, iii) the culture of the invention, iv) the inactivated cell of the invention, v) the spore of the invention or vi) the composition of the invention is applied to a seed or the surroundings thereof.

The strain of the invention is also capable of promoting growth in a plant, where plant growth can refer to the increase in plant length, the increase in size or length of the stem(s), increase in root length, increase in dry weight of the plant and/or increase in water retention in plant tissues, where the increases in the aforementioned parameters can be at least 1.1 times, 1.2 times, 1.5 times, 1.7 times, 2 times, 2.5 times, 3 times, 4 times or 5 times higher in plants to which the strain of the invention has been applied compared to plants to which the strain of the invention has not been applied.

Furthermore, the strain of the invention presented the ability to solubilise phosphate and urease activity, which benefit the absorption of nutrients such as phosphate and nitrogen from urea by the plant. It also presented other enzymatic activities relevant to the cellular function of the plant, such as cytochrome C oxidase and catalase activity. It was also capable of producing phytohormones and different metabolites which are involved in other functions of the plant (table 3 of example 7).

In another particular embodiment, the mutant of the invention also maintains the following capabilities:
- phosphate solubilisation;
- urease activity;
- catalase activity; and/or
- cytochrome oxidase activity.

Regarding the effects related to the development of a plant, the strain of the invention showed promotion of flowering. In the present invention the terms "flowering" or "blooming", used interchangeably, refer to the physiological process by which a plant generates the reproductive organ called a flower, the strain of the invention is thereby capable of promoting the formation of flowers in a plant or in a population of plants. Therefore, "promote flowering in a plant" refers to shortening the period from when the plant germinates until the plant flowers for the first time, to increasing the number of flowers produced per plant and/or increasing the proportion of plants that flower in a given plant population.

Surprisingly, the strain of the invention is capable of modifying the soil microbiota. More specifically, the strain of the invention is capable of increasing the proportion of plant growth-promoting rhizobacteria in the soil, in other words, increases the proportion of plant growth-promoting rhizobacteria in the total proportion of microorganisms that make up the soil microbiota. The term "plant growth-promoting rhizobacteria (PGPR)" refers to bacteria that can increase the growth and productivity of a plant, which live mainly in the rhizosphere. They are bacteria capable of producing hormones and metabolites beneficial to plants. Examples of PGPRs include, but are not limited to, bacteria that belong to the family Gemmatinomonadaceae, Rubrobacteriadaceae, Nitrosomonadaceae, Sphingomonadaceae and the family Bacillaceae.

In preferred embodiments, the PGPRs that increase their proportion in the soil are bacteria that belong to the Gemmatinomonadaceae family, Rubrobacteriadaceae, Nitrosomonadaceae, Sphingomonadaceae or the family Bacillaceae, preferably, where the bacteria that belong to the family Sphingomonadaceae are of the genus, *Sphingomonas, Sphingobium, Novosphingobium or Sphingopyxis;* more preferably, *Sphingomonas panaciterrae or Sphinogomonas sediminicola.*

The above-mentioned capacities can be determined by carrying out the corresponding tests as in the example section of the invention.

Furthermore, the strain of the invention was also capable of exerting control over pathogenic microorganisms in a plant.

In the present invention, the term "control of pathogenic microorganisms" refers to, reducing, inhibiting, preventing accumulation and/or elimination of pathogenic microorganisms, preferably in a plant or population of plants.

In the present invention, the term "pathogenic microorganisms of a plant" refers to a microorganism capable of producing a disease or pathological state in a plant related to a deterioration of the plant or even the death thereof. Plant pathogenic microorganisms include bacteria (microorganisms belonging to the kingdom Eukarya) or fungi (microorganisms belonging to the kingdom Fungi).

Therefore, in a preferred embodiment of the improvement method of the invention, pathogenic microorganisms are bacteria or fungi.

Specifically, the strain of the invention was capable of controlling pathogenic fungi of a plant, and also presented antibacterial effect.

Examples of plant pathogenic fungi include, but are not limited to, fungi that belong to the genus *Botrytis* or *Fusarium.*

In another particular embodiment of the improvement method of the invention, the pathogenic fungus is *Botrytis cinerea* or *Fusarium oxysporum.*

In the present invention, the term "plant" refers to any living organism belonging to the kingdom Plantae, preferably any living organism belonging to any genus/species of the kingdom Plantae. The plant can be at any stage of development: seed, seedling, young plant or adult plant (with the capacity to produce fruit).

Due to its characteristics, the strain of the invention can be used in the field of agriculture in order to improve the performance and/or characteristics of crop plants. Therefore, the term plant preferably refers to a crop plant.

In the present invention, the term "crop plant" refers to any plant that is cultivated by humans for ornamental purposes or to obtain a product of interest therefrom (for example, to obtain fruits, tubers or a part of the plant such as the flower or its leaves).

Examples of plants, preferably crop plants, include, but are not limited to, horticultural plants, woody plants, extensive crop plants, fruit plants, forage plants or oil plants. More specifically, examples of crop plants include, but are not limited to, pepper, cucumber, cress, olive, rice, apple, wheat, kiwi, corn, barley, millet, sorghum, oat, soybean, rye, alfalfa, sunflower, tobacco, potato, cotton, sweet potato, yucca, coffee, coconut, pineapple, citrus, cacao, tea, banana, avocado, figs, guava, mango, papaya, cashew, macadamia, almond, sugar beet, sugar cane, tomato, lettuce, pea, cucurbit, cherry, grapevine, cucumber, conifer and grass plants.

In the present invention the term "plant part" refers to any part of a plant, including, but not limited to the bud, root, stem, seeds, stipules, leaves, petals, flowers, ovules, bracts, branches, petioles, internodes, bark, wood, tubers, pubescence, shoots, rhizomes, fronds, leaves, pollen, stamen, microspores, fruits and seeds.

The two main parts of the plants cultivated in typical media used in the art, such as soil, are usually referred to as the "aerial" part, also referred to as "buds", and the "underground" part, which is also referred to as "roots".

Therefore, in another particular embodiment of the improvement method of the invention, the plant is a crop plant, preferably selected from the list consisting of a horticultural plant, woody plant, extensive crop plant, fruit plant, forage plant and oil plant. More specifically, examples of crop plants include, but are not limited to, pepper, cucumber, cress, olive, rice, apple, wheat, kiwi, corn, barley, millet, sorghum, oat, soybean, rye, alfalfa, sunflower, tobacco, potato, cotton, sweet potato, yucca, coffee, coconut, pineapple, citrus, cacao, tea, banana, avocado, figs, guava, mango, papaya, cashew, macadamia, almond, sugar beet, sugar cane, tomato, lettuce, pea, cucurbit, cherry, grapevine, cucumber, conifer and grass plants.

In the present invention, the term "plant surroundings" refers to the environment or material in which the plant is located, preferably where the surroundings of the plant comprise the conditions and/or elements for the survival of the plant. More preferably, the surroundings of the plant refer to the substrate in which the plant is placed, more specifically where the underground part of the plant is located. Therefore, the surroundings of the plant preferably refer to the earth, soil, compost, mulch or plant growing medium, in which the plant is located, preferably in which the underground part of the plant is located. The surroundings of the plant may comprise a perimeter in the form of a circle with a radius of at least 10 metres, preferably 9, 8, 7, 6, 5, 4, 3, 2 metres or 1 metre. The surroundings of a plant may include other elements such as leaf litter, sawdust, straw, pine straw, wood chips, gravel or other material in a bed surrounding the plant.

Therefore, in another preferred embodiment of the improvement method of the invention, the strain of the invention i), the mutant of the invention ii), the culture of the invention iii), the inactivated cell of the invention iv), the spore of the invention v) or the composition of the invention vi), is applied to the rhizosphere, phyllosphere and/or endosphere of the plant.

Therefore, in another more preferred embodiment of the improvement method of the invention, the strain of the invention i), the mutant of the invention ii), the culture of the invention iii), the inactivated cell of the invention iv), the spore of the invention v) or the composition of the invention vi), is applied to:
- the roots, leaves, fruits, flowers, calluses, tubers, stems or seeds of the plant or any combination thereof, and/or
- earth, soil, compost, mulch, leaf litter, sawdust, straw, pine straw, wood chips, gravel, plant growing medium, other material in a bed surrounding the plant, or any combination thereof.

In the context of the present invention, the term "apply" refers to contacting the plant, part of the plant or surroundings of the plant with the strain of the invention i), the mutant of the invention ii), the culture of the invention iii), the inactivated cell of the invention iv), the spore of the invention v) or the composition of the invention vi). The person skilled in the art knows the methods for applying compounds to a plant, part of a plant or surroundings of a plant, such as spraying, spreading, by means of irrigation water or mixing with the earth, carriers, soil or compost where the plant is located.

In another preferred embodiment of the improvement method of the invention, the strain of the invention i), the mutant of the invention ii), the culture of the invention iii), the inactivated cell iv), the spore v) or the composition of the invention vi) are formulated as a solid formulation, liquid formulation, granules, powder, wettable powder, suspension concentrate or emulsifiable concentrate.

According to improvement method of the invention, the strain of the invention i), the mutant of the invention ii), the culture of the invention iii), the inactivated cell iv), the spore v) or the composition of the invention vi) is administered in an effective amount.

In the present invention, the term "effective amount" refers in the present invention to the amount of the strain of the invention, the mutant of the invention, the crop of the invention or the composition of the invention that when applied to the plant produces the intended effect without producing a toxic effect. In the context of the present invention, the intended effect is to improve the resistance of a plant to drought, promote germination in a plant, promote the growth of a plant, control pathogenic fungi in a plant, reduce the water supply to a plant (that is, reduce the irrigation needs for the plant), promote flowering in a plant and/or increase the proportion of plant growth-promoting rhizobacteria in the soil where a plant is grown. As is evident to a person skilled in the art, effective amounts may vary depending on several factors such as, for example, the type of plant, the physiological condition of the plant, weather conditions (for example, temperature, humidity) or the growth phase of the plant. The person skilled in the art knows how to calculate the effective amount to be applied based on the aforementioned factors or other factors.

In another aspect, they refer to a use, hereinafter, "use of the invention", of:
i) the strain of the invention,
ii) the mutant of the invention,
iii) the culture of the invention,
iv) the inactivated cell of the invention,
v) the spore of the invention, or
vi) the composition of the invention,
for
- improving the resistance of a plant to drought,
- promoting the germination in a plant,
- promoting the growth of a plant,
- the control of pathogenic microorganisms in a plant,
- reducing the water supply to a plant,
- promoting flowering of a plant, and/or
- increasing the proportion of plant growth-promoting rhizobacteria in the soil where a plant is grown.

In a preferred embodiment of the use of the invention, the plant is a crop plant, preferably selected from the list consisting of a horticultural plant, woody plant, extensive crop plant, fruit plant, forage plant and oil plant; more preferably the crop plant is selected from the list consisting of pepper, cucumber, cress, olive, rice, apple, wheat, kiwi, corn, barley, millet, sorghum, oat, soybean, rye, alfalfa, sunflower, tobacco, potato, cotton, sweet potato, yucca, coffee, coconut, pineapple, citrus, cacao, tea, banana, avocado, figs, guava, mango, papaya, cashew, macadamia, almond, sugar beet, sugar cane, tomato, lettuce, pea, cucurbit, cherry, grapevine, cucumber, conifer and grass plants.

In a preferred embodiment of the use of the invention, promoting germination is a plant in the seed state, therefore, preferably i) the strain of the invention, ii) the mutant of the invention, iii) the culture of the invention, iv) the inactivated cell of the invention, v) the spore of the invention or vi) the composition of the invention is applied to a seed or the surroundings thereof.

In another preferred embodiment of the use of the invention, pathogenic microorganisms are bacteria or fungi.

In another particular embodiment of the use of the invention, the pathogenic fungus is *Botrytis cinerea* or *Fusarium oxysporum.*

The use of the invention related to the control of pathogenic microorganisms can be formulated as a phytosanitary use or as a pesticide for the strain of the invention, the mutant of the invention, the culture of the invention or the composition of the invention.

In another aspect, the present invention relates to a plant or part of a plant, hereinafter "the plant or part of a plant of the invention", comprising:
i) the strain of the invention,
ii) the mutant of the invention,
iii) the culture of the invention,
iv) the inactivated cell of the invention,
v) the spore of the invention, or
vi) the composition of the invention.

The plant or part of a plant of the invention may comprise i) the strain of the invention, ii) the mutant of the invention, iii) the culture of the invention, iv) the inactivated cell of the invention, v) the spore of the invention or vi) the composition of the invention, on its surface or within its tissues or cells.

Therefore, in a particular embodiment of the plant or part of a plant of the invention, the term "comprises" refers to the fact that the plant or part of the plant is coated or inoculated with i) the strain of the invention, ii) the mutant of the invention, iii) the culture of the invention, iv) the inactivated cell of the invention, v) the spore of the invention or vi) the composition of the invention, on its surface or within its tissues or cells.

In the present invention, the term "coated" refers to the fact that the plant or part of the plant is completely or partially covered on its surface by i) the strain of the invention, ii) the mutant of the invention, iii) the culture of the invention, iv) the inactivated cell of the invention, v) the spore of the invention or vi) the composition of the invention, on its surface or within its tissues or cells.

In the present invention, the term "inoculated" refers to the fact that the plant or part of the plant has been put into contact with the strain of the invention, ii) the mutant of the invention, iii) the culture of the invention, iv) the inactivated cell of the invention, v) the spore of the invention or vi) the composition of the invention, on the surface thereof or within the tissues or cells thereof, such that the plant or part of the plant comprises on the surface thereof, in the tissues or in the cells thereof the strain of the invention, the mutant of the invention or the strain of the invention.

In another particular embodiment of the plant or part of a plant of the invention, the part is selected from the list consisting of the root, leaf, fruit, flower, callus, tuber, stem and seed of the plant; preferably where the part of the plant is an isolated part, where "isolated" refers to part of the plant that has been separated from the plant from which it came.

In another particular embodiment of the plant or part of a plant of the invention, the plant is a crop plant, preferably selected from the list consisting of a horticultural plant, woody plant, extensive crop plant, fruit plant, forage plant and oil plant; more preferably the plant is selected from the list consisting of pepper, cucumber, cress, olive, rice, apple, wheat, kiwi, corn, barley, millet, sorghum, oat, soybean, rye, alfalfa, sunflower, tobacco, potato, cotton, sweet potato, yucca, coffee, coconut, pineapple, citrus, cacao, tea, banana, avocado, figs, guava, mango, papaya, cashew, macadamia, almond, sugar beet, sugar cane, tomato, lettuce, pea, cucurbit, cherry, grapevine, cucumber, conifer and grass plants.

Regarding use in agriculture, it is important to obtain large quantities of cells of the strain or mutant of the invention.

Therefore, another aspect the present invention relates to a method for obtaining a cell culture, hereinafter "the culturing method of the invention", wherein said method comprises:
(a) inoculating the strain of the invention or the mutant of the invention in a culture medium, and
(b) subjecting the inoculated culture medium from step (a) to conditions suitable for the growth of the strain or mutant.

In a preferred embodiment of the culturing method of the invention, the culture medium is liquid or solid.

In the present invention, the culture medium includes the necessary elements for the bacteria grown therein can replicate and/or carry out their metabolic function, thus, the culture medium comprises at least one carbon source (for example, glucose, saccharose or fructose), a nitrogen source (peptone, meat extract, yeast extract or soy extract), a phosphorus source (for example, calcium phosphate, di-potassium or hydrogen phosphate) and other elements such as iron or magnesium.

In another preferred embodiment of the culturing method of the invention, the culture medium is selected from the list consisting of Luria-Bertani (LB) medium, Tripto-Casein Soy Broth (TSB), Nutrient Broth (NB) or M9 minimal medium. Suitable conditions for the growth of the strain or mutant of the invention can be established by the person skilled in the art in a routine manner.

In another preferred embodiment of the culturing method of the invention, suitable growing conditions include a temperature between 25 °C and 35 °C, and a pH value between 6 and 8, preferably with an oxygen concentration between 10 and 50 %.

Once the growth step in the growth method of the invention has been carried out, the culture medium with the cells can be subjected to an isolation or separation step.

Therefore, in a preferred embodiment, the culture method of the invention further comprises a step (c) for separating the strain or mutant of the invention from the culture medium after step (b).

The separation of cells from a culture medium can be carried out by methodology known to the person skilled in the art, such as centrifugation or filtration.

Therefore, in a preferred embodiment of the culturing method of the invention, step (c) is carried out by filtration or centrifugation.

After step (b) or (c) of the culturing method of the invention, an additional preservation step can be carried out by dehydration, condensation or lyophilisation.

The definitions included throughout the description apply to any aspect or preferred embodiment of the present invention.

Throughout the description and the claims, the word "comprises" and its variants do not intend to exclude other technical features, additives, components or steps. Furthermore, the word "comprises" includes the case of "consists of". For those skilled in the art, other objects, advantages and features of the invention may be partially deduced from both the description and the embodiment of the invention. The following examples and drawings are provided by way of illustration and are not intended to limit the present invention. Furthermore, the present invention covers all possible combinations of particular and preferred embodiments indicated herein.

### DESCRIPTION OF THE FIGURES

**Figure 1****. Petri dishes with SMRS 1 medium with five drops of bacterial suspension, incubated for 24 hours at 30 °C**. The halos formed by the phosphate solubilising activity are observed with the colour change to yellow when the medium acidifies. A: the plates of strain A6 are used as an example. B: positive control *Pseudomonas putida* KT2440. C: negative control *Arthrobacter koreensis* 5J12A.
**Figure 2. A****) Amount of tricalcium phosphate solubilised by five strains of the genus *Bacillus,* expressed in mg/ml.** The bacterial strains were incubated in Pikovskaya culture medium for 6 days at 30 °C and 180 rpm. The error of each measurement was calculated with the standard deviation of the results when performing the test in duplicate. The *Arthrobacter koreensis* 5J12A strain was used as a negative control and the *Pseudomonas putida* KT2440 strain as a positive control. B) **Standard curve that relates the amount of soluble monopotassium phosphate (µg/ml) to the absorbance (λ 820nm).** Monopotassium phosphate was dissolved in the Pikovskaya culture medium at concentrations from 0 to 10 mM and the same process as the samples was followed to record its absorbance at 820 nm.
**Figure 3. A****) Synthesis of indoleacetic acid (IAA) by strains of the genus *Bacillus* in µg/ml.** IAA production by strains of the genus *Bacillus* studied: DSM7, FZB42, D747, Bc1, A6 and Bc3 through modifications of the method described by Bano and Musarrat 2003. The *Pseudomonas putida* KT2440 and *Arthrobacter koreensis* 5J12A strains were used as positive controls. The error bars were calculated with the standard deviation of the three data collected in the three tests carried out. **B) Standard curve that relates the amount of indoleacetic acid (µg/ml) to the absorbance (λ353nm).** Concentrations of 0 to 10 µg/ml of this compound dissolved in the VitaNGrow culture medium were used and the same process as the samples was followed to record their absorbance at 353 nm.
**Figure 4****. Phytohormone production.** A) Indole-3-acetic acid, B) Indole butyric acid, C) Gibberellic Acid.
**Figure 5****. Photographic and graphic representation of the Italian green pepper tray.** A) Pepper plants in each condition recently extracted from the substrate. B) Graphic representation of the average length of the stem. Time zero is represented in white and time 32 days is represented in grey. Significant differences are indicated by the presence of a letter at time 32 days

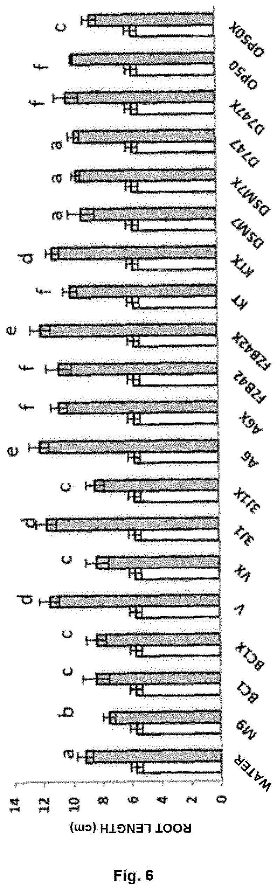

**Figure 6****. Size of the root of the Italian green pepper inoculated with *Bacillus* strains.** Graphic representation of the average length of the root (cm). Time zero is represented in white and time 32 days is represented in grey. Significant differences are indicated by the presence of a letter at time 32 days.
**Figure 7****. Dry weight of Italian green pepper plants inoculated with *Bacillus* strains.** Graphic representation of the average dry weight of the plant (g). Time zero is represented in white and time 32 days is represented in grey. Significant differences are indicated by the presence of a letter at time 32 days.

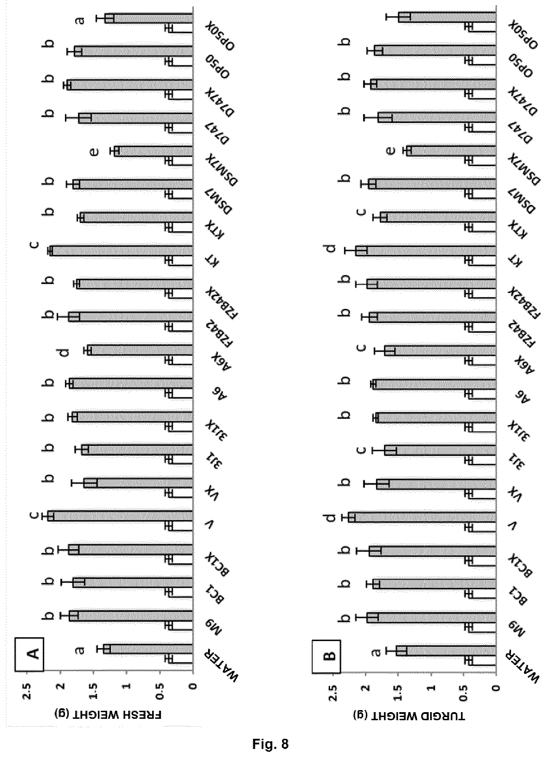

**Figure 8****. Fresh and turgid weights of Italian green pepper plants inoculated with *Bacillus* strains. A)** Graphic representation of the average fresh weight of the plant. **B)** Graphic representation of the average turgid weight of the plant (g). Time zero is represented in white and time 32 days is represented in grey. Significant differences are indicated by the presence of a letter at time 32 days.

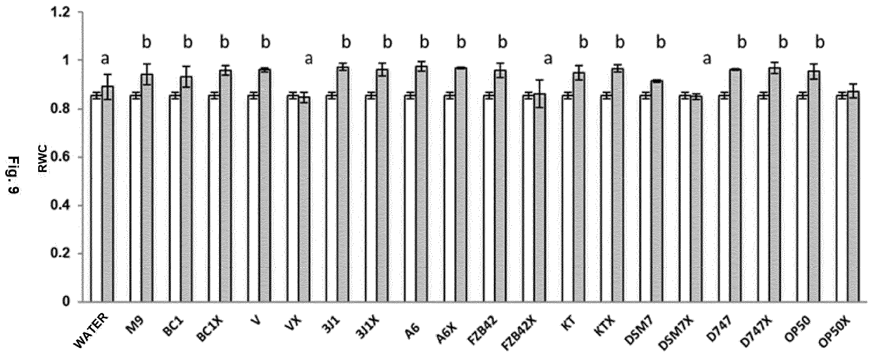

**Figure 9****. Relative Water Content of Italian green pepper plants inoculated with *Bacillus* strains.** Graphic representation of the relative water content (RWC) in the plant. Time zero is represented in white and time 32 days is represented in grey. Significant differences are indicated by the presence of one letter at time zero and two letters at time 32 days.

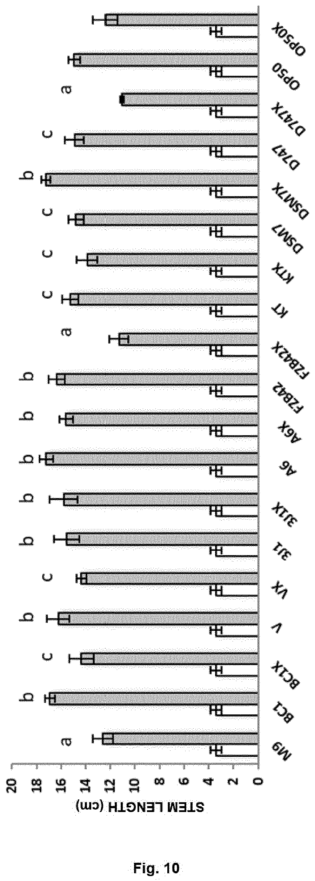

**Figure 10****. Size of the stem of Raf tomato plants inoculated with *Bacillus* strains.** Graphic representation of the average length of the stem. Time zero is represented in white and time 31 days is represented in grey. Significant differences are indicated by the presence of a letter at time 31 days.

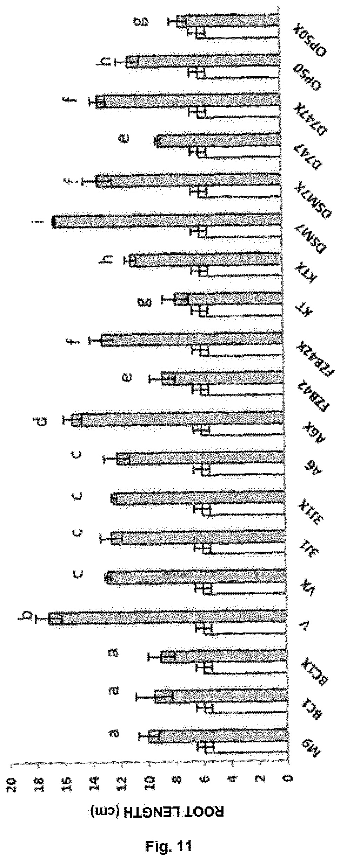

**Figure 11****. Size of the root of Raf tomato plants. Graphic representation of the average length of the root (cm).** Time zero is represented in white and time 31 days is represented in grey. Significant differences are indicated by the presence of a letter at 31 days.

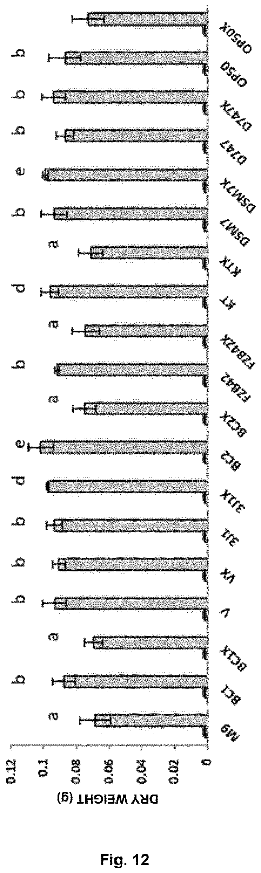

**Figure 12****. Dry weight of Raf tomato plants inoculated with *Bacillus* strains. Graphic representation of the average dry weight of the plant (g).** Time zero is represented in white and time 31 days is represented in grey. Significant differences are indicated by the presence of one letter at time zero and two letters at time 31 days.

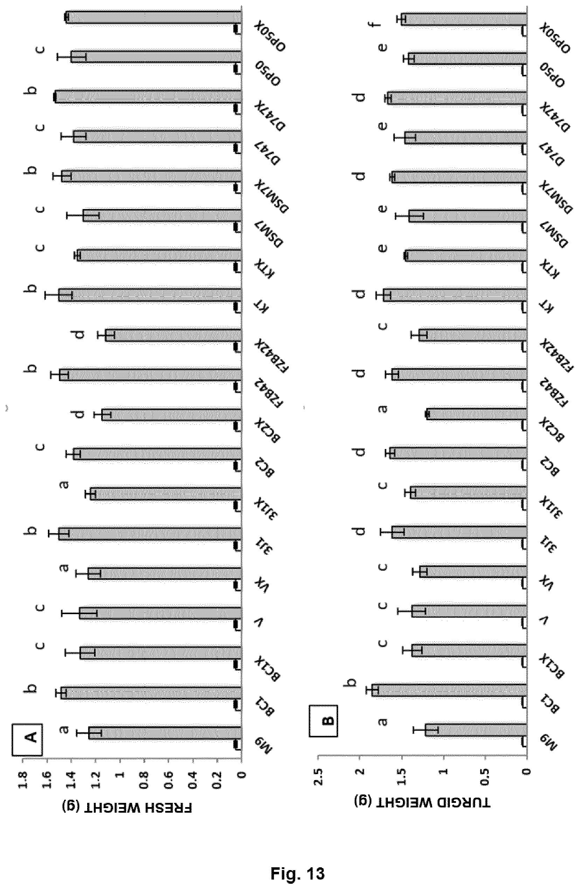

**Figure 13****. Fresh and turgid weight of Raf tomato plants inoculated with different strains of the genus *Bacillus.* A)** Graphic representation of the average fresh weight of the plant. **B)** Graphic representation of the average turgid weight of the plant (g). Time zero is represented in white and time 31 days is represented in grey. Significant differences are indicated by the presence of a letter at time 31 days.

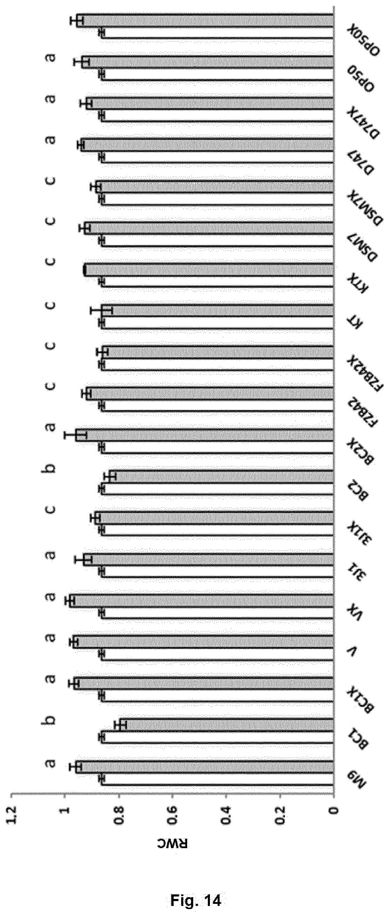

**Figure 14****. Relative Water Content of Raf tomato plants inoculated with strains of the genus *Bacillus.*** Graphic representation of the relative water content (RWC) in the plant. Time zero is represented in white and time 31 days is represented in grey. Significant differences are indicated by the presence of a letter at time 31 days.
**Figure 15****. Germination index of cress seeds inoculated with different *Bacillus.*** The germination index of each bacterium in cress seeds is shown. Significant differences are indicated by the presence of a letter.
**Figure 16. A****) Photograph of the antagonism test of the strains of the genus *Bacillus* on *F. oxysporum.*** The growth of the fungus *F. oxysporum* is shown in the presence of the strains *Bacillus velezensis* A6, *Bacillus velezensis* FZB42, *Bacillus amyloliquefaciens* subsp. *amyloliquefaciens Fukumoto, B. velezensis* BC1 and *Bacillus amyloliquefaciens* D747 *Southerm* at 5, 10, 15 days. **B) Antagonism of the strains of the genus *Bacillus* on *F. oxysporum.*** The growth of the diameter of the fungus *F*. *oxisporum* is shown in the presence of the different *Bacillus* strains at day 5 (white), at day 10 (grey) and at day 15 (black). Statistical assays that are represented by a letter, corresponds to the time 5 days, with two letters at a time 10 days, and with three letters, at the time 15 days.

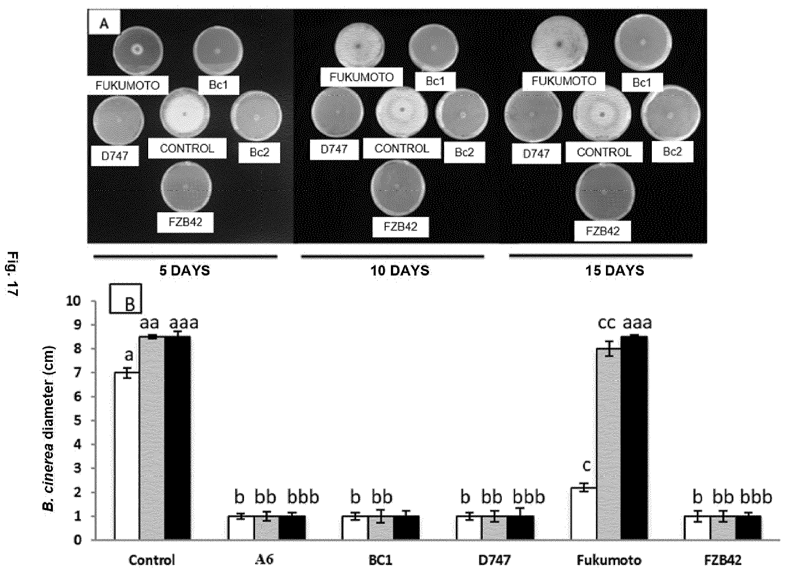

**Figure 17. A****) Photograph of the antagonism test of the strains of the *Bacillus* genus on B. *cinerea.*** The growth of the fungus *F. oxysporum* is shown in the presence of the strains *Bacillus velezensis* A6, *Bacillus velezensis* FZB42, *Bacillus amyloliquefaciens* subsp. *amyloliquefaciens Fukumoto, B. velezensis* BC1 and *Bacillus amyloliquefaciens* D747 *Southerm* at 5, 10, 15 days. **B) Antagonism of the strains of the genus *Bacillus* on B. *cinerea.*** The growth of the diameter of the fungus *F*. *oxysporum* is shown in the presence of the different *Bacillus* strains at day 5 (white), at day 10 (grey) and at day 15 (black).

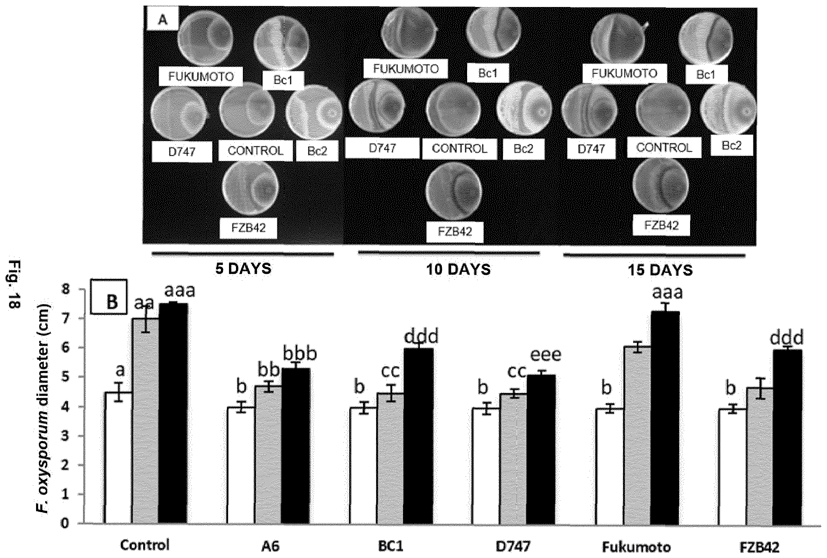

**Figure 18****. Photograph of the antibiosis test of the strains of the genus *Bacillus* on *F. oxysporum.*** The growth of the fungus *F. oxysporum* is shown in the presence of the strains *Bacillus velezensis* A6, *Bacillus velezensis* FZB42, *Bacillus amyloliquefaciens* subsp. *amyloliquefaciens Fukumoto, B. velezensis* BC1 and *Bacillus amyloliquefaciens* D747 *Southerm* at 5, 10, 15 days. **B) Antibiosis of the strains of the genus *Bacillus* on *F. oxysporum.*** The growth of the diameter of the fungus is *F. oxysporum* shown in the presence of the different *Bacillus* strains at day 5 (white), at day 10 (grey) and at day 15 (black).

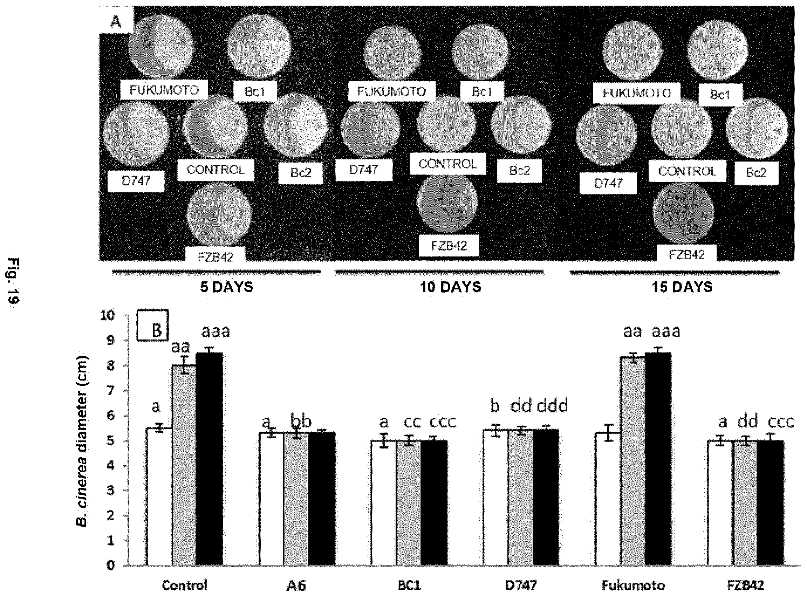

**Figure 19****. Photograph of the antibiosis test of the strains of the genus *Bacillus* on *B. cinerea.*** The growth of the fungus *B*. *cinerea **is shown*** in the presence of the strains *Bacillus velezensis* A6, *Bacillus velezensis* FZB42, *Bacillus amyloliquefaciens* subsp. *amyloliquefaciens Fukumoto, B. velezensis* BC1 and *Bacillus amyloliquefaciens* D747 *Southerm* at 5, 10, 15 days. **B) Antibiosis of the strains of the genus *Bacillus* on B. *cinerea.*** The growth of the diameter of the fungus 8. *cinerea* is shown in the presence of the different *Bacillus* strains at day 5 (white), at day 10 (grey) and at day 15 (black).
**Figure 20.** A
) biostimulation of plants treated with *Bacillus* strains. B) Relative Water Content (CRA) in plants treated with *Bacillus* strains.
**Figure 21****.** Assay of the cytochrome C oxidase enzyme in different strains of the genus *Bacillus* (A6, DSM7, FZB42, D747, Bc1 and Bc3). The *E. coli* HB101 strain was used as a positive control and the *Staphylococcus aureus* CECT 86 strain was used as a negative control.
**Figure 22****. Qualitative assay for the detection of the catalase enzyme in six strains of the genus *Bacillus* (A6, DSM7, FZB42, D747, Bc1 and Bc3).** The *Escherichia coli* HB101 strain is used as a positive control and *Enterococcus faecalis* JH2-2 is used as a negative control. The appearance of bubbles shows positive results for this enzyme.
**Figure 23****. Effects of the application of A6 on the number of flowers per plant, the number of buds per plant and the number of fallen flowers per plant in potato crop.** Data are represented as mean ±SD (n=10). Groups with different letters differ statistically (p<0.05).
**Figure 24****. Effects of the application of A6 on the flowering % in the potato crop.** Data are represented as mean ±SD (n=10). Groups with different letters differ statistically (p<0.05).
**Figure 25****.** Antimicrobial effect of the filtrate of the A6 strain culture.
**Figure 26****.** Effect on plant growth-promoting rhizobacteria.

### EXAMPLES

Next, the invention will be illustrated by means of assays carried out by the inventors that highlight the advantages/effects of the strain of the invention, specifically in the field of agriculture.

Comparative data of the strain of the invention with other strains of the genus *Bacillus* are included. Specifically, the strains used in the experiments were: A6 strain *(Bacillus velezensis* CECT30599), *Bacillus sp.* V (strain isolated from a soil sample from the same location where the strain of the invention was isolated), *Microbacterium sp* 3J1, *Bacillus amyloliquefaciens* subsp. *amyloliquefaciens Fukumoto* DSM7, *Bacillus velezensis* FZB42, *Bacillus amyloliquefaciens* D747, *E. coli* OP50, *Bacillus velezensis* BC1, *Bacillus velezensis* Bc2, *Bacillus velezensis* Bc3, *Arthrobacter koreensis* 5J12A, *Pseudomonas putida* KT2440 or (KT).

Furthermore, comparative data are included using the above strains inactivated by autoclaving at 121 °C, for 30 minutes. Inactivated strains are represented throughout the description and figures as A6X, VX, 3J1 X, DSM7X, FZB42X, D747X, OP50X, Bc1X, Bc2X, Bc3X and KTX.

Throughout the examples and figures, the strains and inactivated strains mentioned above are referred to with the references cited above.

### Example 1. Plant growth promoting properties (PGPRs)

### 1.1. Solubilisation of phosphorus and potassium

In this section, the capacity of the different *Bacillus* strains for the solubilisation of phosphates is studied.

### 1.1.1 Qualitative assay

For the qualitative study of phosphate solubilisation of five *Bacillus* strains, plates were used with SMRS 1 medium that contains a pH indicator that, with the acidification of the medium due to the solubilisation of phosphates, turns yellow. Two plates per strain were prepared and five drops of 20 µl of the bacterial suspension formed by resuspending a colony in 1 ml of 1xM9 were added to each one. These plates were incubated for 24 hours at 30 °C and the diameters of the halos formed by the phosphate solubilising activity were measured, yellow in colour due to the change in the pH of the medium (Table 1 and Figure 1). Sterile medium, free from cells (M9)

**Table 1. Diameter in mm of the solubilisation halos formed on the SMRS 1 medium plates, due to the effect of the pH change caused by the bacterial strains when solubilising the phosphate. The controls used were Pseudomonas putida KT2440 as positive control and Arthrobacter koreensis 5J12A as negative control.**

| | A6 | DSM7 | FZB42 | D747 | BC1 | Bc3 | KT | 5J12A |
|---|---|---|---|---|---|---|---|---|
| Diameter (mm) | 15.6 ± 0.5 | 13 ± 0.5 | 16.3 ± 0.5 | 15.4 ± 0.5 | 16.3 ± 0.5 | 15.5 ± 0.5 | 26.2 ± 0.4 | 0 |

With the results obtained in this assay it was possible to affirm that the different strains of the genus *Bacillus* studied have the capacity to solubilise phosphates (Table 1 and Figure 1). All of them formed a yellow halo in the SMRS 1 medium corresponding to its acidification due to the presence of soluble phosphate. Furthermore, the strain was used *Pseudomonas putida* KT2440 as a positive control which formed the largest solubilisation halos, measuring 26 mm. Moreover, *Arthrobacter koreensis* 5J12A was used as a negative control, which did not form solubilisation halos since it is incapable of solubilising phosphate.

### 1.1.2. Quantitative assay

With the positive results for the qualitative phosphate solubilization test for the different *Bacillus* strains, an assay was carried out for the quantitative study of this phenotype. This assay was repeated twice to obtain more reliable results and calculate the error of each measurement.

Each bacterial strain of the genus *Bacillus* was cultured in 50 ml of Pikovskaya culture medium at 30 °C and 180 rpm for 6 days. The soluble phosphate present in the supernatant of each culture was measured to calculate the amount of phosphate solubilised by the bacteria. 500 µl of the supernatant were isolated, centrifuging the cultures at 12,000 rpm for 5 minutes, to which 500 µl of trichloroacetic acid and 4 ml of the colour reagent were added. The samples were incubated for 15 minutes at room temperature and the absorbance of each one was measured, at a wavelength of 820 nm. To calculate the amount of soluble phosphate in each case, a monopotassium phosphate standard curve was used (Figure 2B). The obtained results are shown in Figure 2A.

After calculation with the standard curve, the amount of soluble phosphate generated by each bacterial strain showed that the *Bacillus* strains studied had the capacity to solubilise phosphate. The strain *P. putida* KT2440, used as a positive control, was the one that solubilised the greatest amount of phosphate (1.92 mg/ml), while the strain A. *koreensis* 5J12A used as a negative control was not able to solubilise the phosphate in the medium.

### 1.2 Phytohormone production capacity

### 1.2.1. Indoleacetic acid production

Indoleacetic acid (IAA) is a natural auxin synthesised in most plants that promotes the differentiation of plant tissues, cell elongation and division and the plant's response to light and gravity. Various studies have shown that indoleacetic acid molecules produced by PGPR have a determining role in plant growth, promoting root and foliar growth of the plant. The application of these molecules in agriculture is very common, chemically synthesising said molecule and applying it to fields as a fertiliser. Currently, the production of indoleacetic acid by PGPR is highly valued to create natural biofertilisers to apply to fields and achieve a more environmentally-friendly agriculture.

To determine the amount of indoleacetic acid that each bacterial strain is capable of synthesising, the method described by Bano and Musarrat was followed (Bano, et al. 2003, Curr. Microbiol. 46, 324-328.) with slight variations. This assay consists of the detection and quantification of the production of indoleacetic acid by each bacterial strain. To do so, the absorbance (λ₅₃₅ₙₘ) of the supernatant of bacterial cultures grown in specific media was measured.

The bacterial strains were cultured in TSB medium, to obtain biomass. After 24 hours at 30 °C and 180 rpm, 2 washes were performed with 0.8% NaCl to remove supplements from the previous medium, and the bacterial strains were re-cultured in VitaNGrow medium in a 1:100 ratio. After incubating these new cultures for 12 hours under the same conditions, the supernatant was used to calculate the concentration of indoleacetic acid synthesised by each bacterial strain. For this purpose, a standard curve was carried out relating the concentration of indoleacetic acid to the absorbance measured at a wavelength of 353 nm, using concentrations from 0 to 10 µg/ml of this compound (Figure 3b). The obtained results are shown in Figure 3a.

By quantifying the production of IAA by the different bacterial strains, it was observed how the different *Bacillus* strains produced very little amount of this phytohormone (between 1 and 2.4 µg IAA/ml). The strain DSM7 produced slightly more IAA compared to the other strains studied (2.4 µg IAA/ml), followed by strain FZB42 which produced 2 µg IAA/ml and lastly, the rest strains of the genus *Bacillus,* which produced around 1 µg IAA/ml. The bacterial strains *Pseudomonas putida* KT2440 and *Arthrobacter koreensis* 5J12A were used as positive controls for this assay, which produced a much higher quantity of IAA (Vilchez, 2016). As expected, strain 5J12A produced almost twice as much phytohormone (18.3 µg IAA/ml) than strain KT2440, which produced 11.6 µg IAA/ml

### 1.2.2. HPLC measurement of indoleacetic acid, indolebutvric acid and gibberellins production

Furthermore, through HPLC, the ability to produce the following was studied:
- Indoleacetic Acid (IAA), as one of the most abundant auxins, which plays a key role in the regulation of various physiological processes such as cell division and elongation, vascular differentiation, gravitropism and phototropism. Among the strains assayed, DSM7 was the highest IAA producer with even higher IAA production (250 µg/l) than the positive control *P. putida* KT2440 (212 µg/l) at 48 hours. The rest of the strains showed a very limited production of IAA in the range of 28-31 µg/l (Figure 4A).
- Indolebutyric Acid (IBA) is another auxin produced by certain bacteria with an important role in plant growth. Similar to IAA production, in the case of the IBA production, DSM7 was the largest producer of this auxin (0.88 µg/l), followed by the other three strains with values ranging between 0.5 (FZB42) and 0.64 µg/l (D747) (Figure 4B).
- Gibberellic acid is also involved in different plant development processes and the regulation of many physiological processes (shah, S.H., Islam, S., Mohammad, F. et al. J Plant Growth Regul 42, 7352-7373 (2023). Once again, DSM7 was the strain with the highest production of the phytohormone. In this specific case, it was the only producer of gibberellic acid with more than 1.23 µg/l, even higher than that recorded for the positive control of *P*. *putida* KT2440 (0.87 µg/l). No production of gibberellic acid was detected in the other *Bacillus* strains (Figure 4C). In all cases, gibberellic acid was only found at 24 hours but not at 72 hours.

### 1.3 Siderophore production capacity

The ability to chelate iron through the production of siderophores is a very important PGPR characteristic, since on the one hand it will improve the transport of this nutrient to the plant, and will also reduce the impact of phytopathogenic fungi in the soil.

In this study, all strains were capable of producing siderophores. Again, strain A6 was positive for this PGPR activity.

### Example 2. Biostimulant properties in different crops

### 2.1 Assay on Italian sweet pepper plants

An additional control was incorporated in this trial, with only water. The results obtained indicate that plants inoculated with bacteria of the genus *Bacillus* used in the assay increased the length of the stem with respect to the controls with water or with M9* buffer added (Figure 5A). The control plants with only water added had an average stem length value of 6.8±0.73 cm and the stem of the control plants supplemented with M9 buffer was 7.6±0.55 cm, while the plants inoculated with the control strains *Microbacterium* sp. 3J1, had an average stem size of 9.88±0.25 cm and those inoculated with *P. putida* KT2440 had an average size of 9.69±0.47 cm. The plants inoculated with the *Bacillus* strains that had the longest stem length were those inoculated with A6, the stem size of which was 10.18±0.47 cm, as well as the plants inoculated with the FZB42 strain, average stem size of which was 9.96±0.43 cm (Figure 5B). *M9 buffer: culture medium without bacteria.

With respect to the values recorded in the length of the root, the control of plants with water added was 9.2±0.54 cm, with M9 buffer it was 7.56±0.38 cm, with *Microbacterium sp.* 3J1 it was 11.68±0.70 cm and with *P*. *putida* KT2440 it was 9.97±0.47 cm. The plants that had a greater root length were those inoculated with the A6 strains with 12.15±0.67 cm, FZB42X with 12±0.85 cm and V with 11.52±0.94 cm (figure 6).

To verify the increase in organic matter gained by plants, their dry weight was recorded. The dry weight values recorded for the plants inoculated with the controls were 0.11±0.01 g for water control, 0.12±0.004 g for plants supplemented with M9 buffer, while the dry weight of plants inoculated with *Microbacterium sp.* 3J1 it was 0.15±0.01 g and for those inoculated with *P*. *putida* KT2440 it was 0.165±0.01 g. The plants inoculated with the *Bacillus* strains that had a higher dry weight were those inoculated with A6 and FZB42, along with V, VX and 3J1X, all weighing 0.17±0.01 g (figure 7).

The fresh weight of the plants indicates the growth of the plant due to the accumulation of both organic matter and water. While those inoculated with water had a fresh weight of 1.35±0.1 g. The plants with M9 buffer added had a fresh weight of 1.86±0.1 g, while those inoculated with 3J1 had a fresh weight of 1.68±0.1 g. In the case of plants inoculated with *P. putida* KT2440, the fresh weight was 2.15±0.03 g. Only plants inoculated with strain V presented a fresh weight greater than the controls with 2.18±0.1 g. The turgid weight indicates the ability of the plant to absorb water in an environment of total hydration. For turgid weight values, plants supplemented with water alone were 1.53±0.2 g. That of the plants supplemented with M9 buffer was 1.98±0.2 g. The plants inoculated with *Microbacterium sp.* 3J1, presented a fully turgid weight (FTW) of 1.71±0.2 g, for those inoculated with *P*. *putida* KT2440 it was 2.16±0.2 g. In this case, as in the case of turgid weight, the highest recorded value corresponded to that of the plants inoculated with the V strains, the FTW of which was 2.26±0.1 g (figure 8A, 8B).

The relative water content (RWC) indicates the hydration state of the plant. It is a dimensionless value, the theoretical maximum of which is 1 in the case of a state of complete hydration by the plant. The RWC value of the control plants was, for those added with water 0.89±0.05, for those supplemented with M9 buffer 0.99±0.04, for those inoculated with the 3J1 strain 0.97±0.02 and for those inoculated with the KT2440 strain 0.95±0.03. Only plants inoculated with strain A6 had a RWC greater than or equal to the controls with M9 0.97±0.02. Furthermore, it was observed that when the dead strains V and FZB42 were used, the RWC was lower than that observed in plants supplemented with M9, but equal to that of plants with water added (figure 9).

As a relevant conclusion of this assay, the importance of the ability of strain A6 not only to improve plant biomass, but to make the water content in plants improve during their development was observed.

### 2.2 Assay on Raf tomato plants

The assay described above was also repeated on Raf tomato plants. Regarding the length of the stem, control tomato plants with only added M9 buffer, it was 12.6±0.8 cm, while the plants inoculated with the control strains *Microbacterium* sp. 3J1, had an average stem size of 15.6±1.03 cm and those inoculated with *P. putida* KT2440 had an average size of 15.3±0.64 cm. The plants inoculated with the *Bacillus* strains that had the longest stem length were those inoculated with A6, the stem size of which was 17.2±0.6 cm, the plants inoculated with the strain BC1 the stem size of which was 16.93±0.4 cm and the plants inoculated with the strain DSM7 the average stem size of which was 7.3±0.6 cm (figure 10).

With respect to the values recorded in the length of the root, the control plants with M9 buffer added was 10±0.7 cm, with *Microbacterium* sp. 3J1 was 12.55±0.8 cm and with *P. putida* KT2440, it was 7.7±1 cm. The plants that had a greater root length were those inoculated with the DSM7 strains with 16.5±0.1 cm, FZB42X with 15.25±0.9 cm and V with 17.1±0.9 cm (figure 11).

To verify the increase in organic matter gained by plants, their dry weight was recorded, the dry weight values recorded for the plants inoculated with the controls were for the control supplemented with M9 buffer 0.07±0.01g, while the dry weight of plants inoculated with *Microbacterium sp.* 3J1 was 0.09±0.005 g and for those inoculated with *P. putida* KT2440 was 0.1±0.005 g. The plant inoculated with the *Bacillus* strain that had a higher dry weight was that inoculated with A6 with a weight of 0.16±0.007 g (figure 12).

In the case of fresh weight, plants with M9 buffer added resulted a fresh weight of 1.3±0.1 g, while those inoculated with 3J1 and *P. putida* KT2440, showed a fresh weight of 1.5±0.1 g. Only plants inoculated with the strains Bc1, FZB42 and D747X had a fresh weight of 1.5±0.04 g. For turgid weight values, plants supplemented with only M9 buffer were 1.21±0.15 g (figure 13A). The plants inoculated with *Microbacterium sp.* 3J1, presented a fully turgid weight (FTW) of 1.6±0.14 g, for those inoculated with *P. putida* KT2440 it was 1.7±0.09 g. In this case, as in the case of turgid weight, the highest recorded value corresponded to that of the plants inoculated with the strain BC1 with 1.9±0.07 g (Figure 13B).

To analyse the significance of the differences, an ANOVA statistical study was used in the two plant trials with the Statgraphics Centurion 18 program, where it was considered that the means of the stem length, root, dry weight, fresh weight, turgid weight and RWC, were significant when they had a value of *p*≤0.05

Conclusion: the biostimulatory effect of strain A6 is demonstrated in all assays, assessing the water retention capacity in those plants treated with A6.

### Example 3. Improved germination rate

As mentioned above, a study has been carried out on the capabilities of different *Bacillus* in improving germination. The assay carried out is described below:
In the case of seeds incubated in the buffer used for bacterial suspensions (0.5xM9), it was observed that 14.3±2.08 of 20 seeds germinated after 48 hours. With these results, the calculation of the germination index (GI) was 0.28±0.03. When the seeds were incubated in the presence of 10⁷ CFU/ml of the controls considered as plant growth stimulants (*P*. *putida* KT2440 and *Microbacterium* sp. 3J1), a germination was observed of 15±1 of 20 seeds used for the strain of *Pseudomonass* and 17.6±0.58 of the 20 seeds used when the strain present was *Microbacterium* sp. 3J1, which represent GIs of 0.23±0.015 and 0.35±0.011 respectively, highlighting that in the case of 3J1, the GI was higher than the GI observed in the absence of microorganism. The results observed in the presence of each of the strains under study are shown in Table 2. Only when strain A6 was present with the seeds, was a higher GI observed, higher than both the control without bacteria, and the KT2440 control and *Microbacterium* sp. 3J1. The lowest germination values were observed in the presence of strains OP50 and FZB42, which presented values less than 0.1 Gl. In the rest of the strains under study, no significant differences were observed with the GI values observed in seeds incubated with water or with 0.5xM9 buffer. The data were subjected to an ANOVA assay to identify whether the observed differences were significant, which are indicated in figure 15.

**Table 2. Averages of the germination index of the different strains with their standard deviation.**

| **Strains** | **Average GI** | **SD** |
|---|---|---|
| **M9** | 0.27674417 | 0.034863198 |
| **BC1** | 0.193375 | 0.027229191 |
| **A6** | 0.441259259 | 0.03654444 |
| **3J1** | 0.352373188 | 0.011515628 |
| **V** | 0.278139535 | 0.017383721 |
| **KT** | 0.232075472 | 0.015471698 |
| **D747** | 0.202962963 | 0.031687582 |
| **FUKUMOTO** | 0.154375 | 0.027229191 |
| **FZB42** | 0.079761905 | 0 |
| **OP50** | 0.058298611 | 0.005807778 |

To analyse the significance of the differences, an ANOVA statistical study was used in the seed assay with the Statgraphics Centurion 18 program, where it was considered that the average GI was significant when it had a value of *p*≤0.05.

### Example 4. Study of the biocontrol against phytopathogenic fungi

As support to the biostimulation capacity, improvement of germination rate, and crop resistance to drought, the A6 strain to be patented shows a high biocontrol capacity against phytopathogenic fungi. For this, two types of assays were carried out: Antagonism Assay and Antibiosis Assay.

### 4.1. Antagonism Studies

### 4.1.1. Study of antagonism on F. oxysporum

To analyse the effect of antagonism on *F. oxysporum,* the fungus and the *Bacillus* strains studied independently were put together in a Petri dish. As the days passed, the development of the fungus was observed in the presence of the different *Bacillus* strains. As a result of the interaction, it was observed that *F. oxysporum,* at day 5, in the absence of bacteria, spread, occupying 4.8 cm of the plate. At day 10 of the assay, the fungus occupied the rest of the plate (7.5 cm). However, at day 10, the presence of the bacteria B. *amyloliquefaciens* D747 restricted the growth of the fungus to 1.4 cm in diameter and the presence of *B. velezensis* BC1 restricted the growth of the fungus to 1.8 cm. At day 15 of the assay, the diameter of the *F. osysporum* colony was 2 cm when the medium was inoculated with the strain *B. amyloliquefaciens* D747 and 2.2 cm when the inoculated strain was *B. velenzensis* BC1. In the case of the strain *B. velezensis* A6, a similar effect to that of strains D747 and BC1 was observed, where *F. oxysporum* managed to occupy in the same way as D747, the ecological niche of *F. oxysporum,* barely letting the fungus grow, 1.6 cm in diameter at day 5 of the assay, 1.8 cm in diameter at day 10 of the assay and 2.2 cm in diameter at day 15 of the assay. In the presence of the strain *B. velezensis* FZB42, it was observed that the fungus grew up to 2.1 cm in diameter at day 5 of the assay, 3.5 cm in diameter at day 10 of the assay and 5.2 cm at day 15 of the assay, while the presence of the strain *B. amyloliquefaciens Fukumoto,* produced the same effect as the positive control of *F. oxysporum,* where the fungus was 3.2 cm in diameter at day 5 of the assay, 7 cm in diameter at day 10 of the assay and 7.5 cm in diameter by which it occupied the medium of the Petri dish at day 15 of the assay (Figure 16A).

In the results obtained it is observed that the strains *B. velezensis* BC1, *Bacillus velezensis* A6 *and B. amyloliquefaciens* D747, restrict the growth of *F. oxysporum,* while the strains *Bacillus velezensis* FZB42 *and Bacillus amyloliquefaciens* subsp. *Fukumoto* restrict to a lesser degree the growth of *F. oxysporum* (figure 16B).

### 4.1.2. Study of antagonism on B. cinerea

To analyse the effect of antagonism on *B. cinerea,* the fungus and the *Bacillus* strains studied independently were put together in a Petri dish. As the days passed, the development of the fungus was observed in the presence of the different *Bacillus* strains. As a result, it was observed that *B. cinerea,* at day 5 in the absence of bacteria, spread, occupying 7 cm of the plate. At day 10 of the assay, the fungus occupied the rest of the plate (8.5 cm). However, at day 5 the presence of the bacteria *B. amyloliquefaciens* D747 and *B. velenzensis* BC1 restricted the growth of the fungus to 1 cm. In the case of the strains *B. velezensis* A6 and *B. velezensis* FZB42, an effect equal to that of the strains *B. amyloliquefaciens* D747 and *B. velenzensis* BC1 was observed, where the growth of the fungus was restricted from day 5 of the assay. In contrast, the strain *Fukumoto* produced the same effect as the positive control of *B. cinerea,* where the fungus was 2.2 cm in diameter at day 5 of the assay, 8 cm in diameter at day 10 of the assay and 8.5 cm in diameter by which it occupied the medium of the Petri dish at day 15 of the assay (Figure 17 A).

In the results obtained, it can be seen that the strains *B. velezensis* Bc1, *Bacillus velezensis* A6, *B. amyloliquefaciens* D747 and *Bacillus velezensis* FZB42, restrict the growth of *B. cinerea,* preventing it from exceeding cm. Moreover, the strain *Bacillus amyloliquefaciens Fukumoto* does not restrict it (figure 17B).

### 4.2. Study of antifungal activity by antibiosis assay

Antibiosis shows us how a microorganism that reaches an environment that is previously occupied by another microorganism can slow the growth of its competitor by secreting substances such as antibiotics.

### 4.2.1. An antibiosis study on F. oxysporum

To analyse the effect of antibiosis on *F. oxysporum,* the fungus was deposited next to the *Bacillus* bacteria independently, after a few days, the development of the fungus was observed in the presence of the *Bacillus* strains. Once the fungi are inoculated, the control of *F. oxysporum* in the absence of bacteria increased up to 4.5 cm in diameter at day 5, occupying almost the entire plate with 7.5 cm in diameter at day 15 of the assay. On the contrary, with the presence of the bacteria *B. amyloliquefaciens* D747 and B. *velenzensis* BC1, the growth of the fungus was restricted to 4.5 cm in diameter from day 10. In the case of strains *B. velezensis* A6 and *B. velenzensis* FZB42 a similar effect was observed where fungal growth was restricted to 4.7 cm in diameter from day 10 onwards. In contrast, the strain *Fukumoto* had a similar effect to the positive control of *F*. *oxysporum,* where the fungus was 4 cm in diameter at day 5 of the assay, 6.1 cm in diameter at day 10 and 7.5 cm in diameter at day 15. A discolouration was noted in the colour of *F. oxysporum* in the presence of the strains *B. amyloliquefaciens* D747, *B. velenzensis* FZB42 and *B. velezensis* A6, the latter being the most marked (figure 18A).

The results obtained show that the strains *B. velezensis* BC1, *Bacillus velezensis* A6, *B. amyloliquefaciens* D747 and *Bacillus velezensis* FZB42, secrete substances that restrict the growth of *F. oxysporum* (figure 18B).

### 4.2.2. Antibiosis study on B. cinerea

To analyse the effect of antibiosis on *B. cinerea,* the fungus was deposited next to the *Bacillus* bacteria independently, after a few days, the development of the fungus was observed in the presence of the *Bacillus* strains. Once the fungi are inoculated, the control of *B. cinerea* in the absence of bacteria, at day 5, the fungus increased up to 5.5 cm in diameter, occupying almost the entire plate with 8.5 cm in diameter at day 15 of the assay. However, the presence of the bacteria *B. amyloliquefaciens* D747 *and B. velenzensis* BC1 restricted the growth of the fungus to 5.4 cm in diameter from day 5 of the assay. In the case of the strains *B*. *velezensis* A6 and *B*. *velenzensis* FZB42, a similar effect was observed where the growth of the fungus was restricted from day 5 of the assay, letting it grow up to 5.3 cm with *B. velezensis* A6 and 5 cm in diameter with B. *velenzensis* FZB42. On the contrary, the strain *Fukumoto* produced an effect equal to the positive control of *B. cinerea,* where the fungus reached 5.3 cm in diameter at day 5; 8.3 cm in diameter at day 10 and 8.5 cm in diameter at day 15 of the assay (figure 19a).

The results show that the strains B. *velezensis* BC1, *Bacillus velezensis* A6, *B. amyloliquefaciens* D747 and *Bacillus velezensis* FZB42, secrete substances that restrict the growth of *B. cinerea* (figure 19B).

To analyse the significance of the differences, an ANOVA statistical study was used in the antagonism study and the antibiosis study with the Statgraphics Centurion 18 program, where it was considered that the average diameters of the fungi of *F. oxyporum* and *B. cinerea* at day 5, 10 and 15 were significant when they had a value of *p*≤0.05.

### Example 5. Capacity to resist drought

The capacity to protect Italian sweet pepper plants of the four *Bacillus* strains used as agricultural amendments was compared. Strain A6 showed the highest values of fresh weight, dry weight and relative water content at the three sampling times. The capacity of the strains DSM7, FZB42 and D747 to protect pepper plants against drought was similar to each other and was not distinguished from that generated by the control of *P*. *putida* KT2440, unable to protect plants against drought, nor the control in the absence of inoculum.

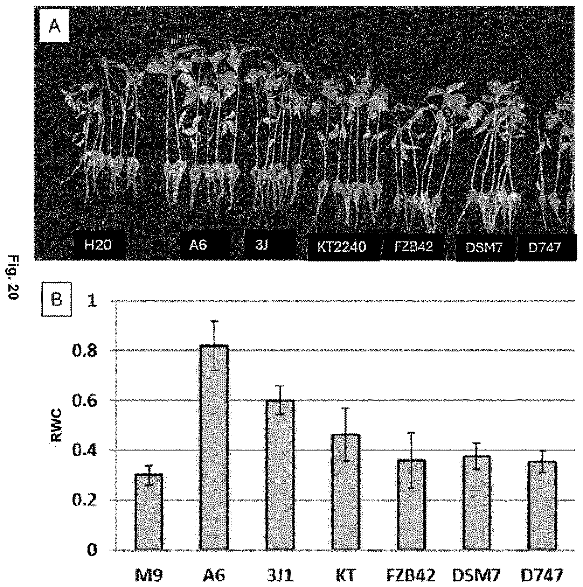

Figure 20a shows the biostimulation of plants treated with the strain A6 compared to the rest of *Bacillus.*

This assay includes an improvement in the capacity to retain water, Figure 20b shows how the plants inoculated with strain A6 showed an increase with a significant difference in the Relative Water Content in the pepper plants upon withdrawal of the assay.

### Example 6. Production of urease enzymes, cytochrome oxidase and catalase

Enzymatic activities play a key role during plant-microorganism symbiosis, and the studies shown below aim to reveal the enzyme production capacity and the differentiation between strains under study. The results allow us to establish that the strain A6 is different from the rest of the strains currently found.

### 6.1 Urease activity

Urea is an important fertiliser for plants as it is a rich source of nitrogen, which is essential for plant metabolism. This compound reaches the soil through the excretions of higher animals and through the destruction of the nitrogenous bases of the nucleic acids present in the remains of plant and animal tissues. The enzyme urease catalyses the hydrolysis of urea, breaking it down into carbon dioxide and ammonium, which is assimilated by plants for the synthesis of proteins and chlorophyll.

In this assay, the urease activity was qualitatively studied in six strains of the genus *Bacillus* (A6, DSM7, FZB42, D747, BC1 and Bc3). To do this, a colony of each bacterial strain was isolated and sown in a test tube with Christensen Urea Agar culture medium and incubated for 24 hours at 30 °C.

This culture medium contains a pH indicator, phenol red. If the bacteria present urease activity and ammonium and carbon dioxide are formed, the pH of the medium becomes alkaline and changes from yellow to red/pink.

The results obtained are shown in Figure 21 where no colour change was observed in the culture medium of the six strains of the genus *Bacillus.* All of them were negative for the qualitative assay of the urease enzyme, so it could be said that they lack this enzyme, just like the negative control *E. coli* MC4100. However, in the case of the positive control *Proteus sp.* S47 it was observed how the colour of the culture medium changed completely, changing from a yellow colour (like the rest of the samples) to a fuchsia pink colour.

### 6.2 Cytochrome oxidase activity

A phenotypic characterisation of the strains of the genus *Bacillus* was carried out using the assay of the cytochrome C oxidase enzyme. This enzyme is located in the electron transport chain of the plasma membrane and catalyses the transfer of electrons from cytochrome C, to the last acceptor which is O₂, producing ATP. Furthermore, it allows distinction between groups of organisms.

This assay was carried out using the reagent N,N,N',N'-tetramethyl-p-phenylenediamine at 1%, which when oxidised by the enzyme cytochrome C oxidase, acquires an intense blue colour. In the event that the bacteria are unable to oxidise it due to not having this enzyme, the reagent remains colourless.

The obtained results are shown in Figure 21, where positive activity of the enzyme cytochrome C oxidase was observed in the strains A6, D747, Bc1 and Bc3, together with the used positive control *E. coli* HB101, since the colonies placed on the filter paper acquire an intense blue colour. In these cases, it can be stated that the oxidase enzyme has oxidised the reagent N,N,N',N'-tetramethyl-p-phenylenediamine, which has given such colouration. However, the strains DSM7 and FZB42 turned out to be negative for this enzyme, as the colonies remained colourless like the negative control used, *Staphylococcus aureus* CECT 86.

### 6.3 Catalase activity

Catalase is an antioxidant enzyme capable of removing reactive oxygen species, in this case H₂O₂ (byproduct of many metabolic reactions), breaking it down into water and oxygen. It is an essential enzyme in the antioxidant system of plants and is found locally in the different organs of the plant. The bacteria that present this activity can be beneficial to complement the antioxidant system of the plant.

Catalase activity was qualitatively detected in six strains of the genus *Bacillus* (A6, DSM7, FZB42, D747, BC1 and Bc3), as described below: two drops of 3% (v/v) hydrogen peroxide were added to an isolated colony of each bacterial strain. In all cases of strains of the genus *Bacillus* bubbles were formed immediately after the addition of hydrogen peroxide, due to the action of the enzyme catalase that degrades hydrogen peroxide into oxygen and water. With these results, all the strains of the genus *Bacillus* were considered positive for the enzyme catalase (Figure 22).

In the case of the selected positive control *E. coli* HB101, bubbles also formed as expected. Lastly, the strain *Enterococcus faecalis* JH2-2 was used as a negative control, which after the addition of hydrogen peroxide did not form bubbles as it lacked the enzyme catalase.

### Example 7. Production of exclusive Secondary Metabolites (SM)

SMs play an important ecological role in defence against microbial pathogens, pests and herbivores, and even competing plants. These are derived from four main chemical classes: polyketides, non-ribosomal peptides, terpenes and indole alkaloids. For this reason, the strain A6 is differentiated from the rest of the patents with microbial strains with a biocontrol effect in which they do not detail the participation of secondary metabolites. Next, the metabolites of the strain A6 and their importance in agriculture are presented.

A study of secondary metabolites exuded after 72 hours of incubation was carried out. A total of 20 antibiotics, 6 antifungals, 8 antioxidants and 2 related to the response to abiotic stress in plants were differentiated.

Of all the metabolites studied, the most representative ones are presented in table 3.

**Table 3. List of secondary metabolites produced by A6.**

| **Secondary metabolite** | **Properties** |
|---|---|
| 1,4-Naphthoquinone | Alkaloid with antibacterial properties |
| Amifloxacin | Antibiotic |
| Clavamycin F | beta-lactam antibiotic |
| Dihydrostreptomycin | Antibiotic |
| G-418 | Aminoglycoside antibiotic |
| Gentamicin X2 | Precursor of the gentamicin C antibiotic |
| Lopinavir | Antiviral |
| Mocimycin | Antibiotic |
| Mycinamycin II | Antibiotic |
| Mycinamycin VII | Antibiotic |
| Niddamycin | Macrolide antibiotic |
| R207910 | Diarylquinolines antibacterial |
| Rimocydin | Macrolide antibiotic |
| Tylosin | Macrolide antibiotic produced by microorganisms |
| Valnemulin | Antibiotic |
| Licoagrodione | Antimicrobial and antibacterial |
| Oleandolide | 14-membered macrolide representing the aglycone of the bacteriostatic antibiotic oleandomycin |
| 1,4-Naphthoquinone | Antibiotic and antifungal properties |
| Ansatrienin A | Antifungal |
| Briotoxin A | Chemopreventive, antitumour, antibacterial and potential insecticide effect |
| Harmalol | Antifungal |
| Ipomeamaronol | Antifungal |
| Marasmene | Antifungal |
| Ramentaceone | Antifungal, antibacterial and cytotoxic agent |
| Spinosyn A | Insecticide |
| Tanacetol A | Natura antioxidant |
| Umbelliprenin | Antifungal |
| 3-Indolebutyric acid | Phytohormones |
| (+)-Chebulic acid | Antioxidant |
| (3Z)-Phycocyanobilin | Antioxidant, anticancer, anti-inflammatory. |
| Acequinocyl | Acaricide |
| Cinobufagin | Anticancer drug |
| Cinobufotalin | Anticancer drug |
| Creatine phosphate | Protects the heart from oxidative stress |
| Vinblastine | Anti-cancer alkaloid |
| Piperidine | Antioxidant |
| Guggulsterone | Prevents oxidation of low-density lipoproteins |
| Panaxynol | Antifungal, antioxidant, anti-inflammatory. |
| Imazaquin | Herbicide |
| Camellianin A | Potential antioxidant activity |
| Cadaverine | Potential growth promoter and modulator against abiotic stress in plants |
| Mocimycin | Antibiotic |
| Mycinamycin II | Antibiotic |
| Picein | Antioxidant |
| Penbutolol | Non-selective beta-blocking drug with intrinsic sympathomimetic properties |
| Pithecolobine | Antimicrobial and antioxidant. |
| Pitheduloside F | Antifungal against post-harvest fungi |
| Feruloylcholine | Involved in response to drought stress |

### Example 8. Effects on flowering and water consumption

The strain of the invention shows the ability to **bring forward flowering** in the harvests, without the plant losing vegetative growth, fruit quality and weight. In order to observe the biostimulant effect of the strain *Bacillus velezensis* A6 was applied to potato plant variety *Spunta.*

For the assay, potato plants were planted in two 2000 m² plots each (sector 1 and sector 2). Both sector 1 and sector 2 were irrigated using drip irrigation. In sector 1, the strain *B. velezensis* A6 was applied using drip water, in three different applications every 15 days.

The flowering capacity was determined at 84 days after sowing, for which 10 sampling points of 1.5 m² were randomly selected for each treatment and the number of flowering plants in this space was quantified, and of every flowering plant, the number of buds and the number of fallen flowers.

The results showed early flowering, the area treated with A6, sector 1, which presented a greater number of flowers per plant (6.28) compared to the untreated area, sector 2 (4.30), highlighting the biostimulant effect of the A6 strain, which allows the plant to improve its flowering without reducing its development. By being favoured by the plant-microorganism symbiosis, it has been able to improve its development and bring forward its vegetative cycle, moving into early flowering. In sector 1, the flowering percentage was significantly higher at 88.93% compared to Sector 2 which confirms that treatment with A6 has a positive impact on the flowering of plants.

Furthermore, the strain to be presented A6 has demonstrated the capacity to reduce irrigation in crops by up to 40% without compromising the crop. Therefore, the strain of the invention presents an improvement in irrigation efficiency.

### Example 9. Antimicrobial activity

When sowing *Microbacterium koreensis* 3J1 and *B. velezensis* A6 on the same plate, it was observed that the presence of A6 colonies prevented the growth of 3J1.

Subsequently, assays were carried out based on the cultivation of three microorganisms until their stationary phase, at which time the cells were separated from the medium by centrifugation, followed by filtration (with 0.020 micron pore size filters).

Three 24-hour cultures were taken and their Abs₆₀₀ were measured, which were:
A6: 2.56
3J1: 1.25
KT: 3.68

They were centrifuged for 10 min at 10,000 rpm and the supernatant was transferred to a new tube and filtered. The cell bud was resuspended in 1xPBS to achieve an Abs of approximately 5 (see table 4 below as Abs_{f}).

**Table 4. Crop absorbance**

| Strain | Absᵢ | Abs_{f} | Added Vol |
|---|---|---|---|
| | | | |
| A6 | 2.56 | 5.83 | 51 uL |
| 3J1 | 1.25 | 5.00 | 60 uL |
| KT | 3.68 | 5.16 | 58 uL |

| | | | |
|---|---|---|---|
| **Absᵢ: initial absorance; Abs_{f}: final absorbance. | | | |

5 ml of fresh LB culture medium was taken and mixed with 1 ml of filtered supernatants, and approximately 50 uL of each culture was added to obtain an initial Abs of 0.05.

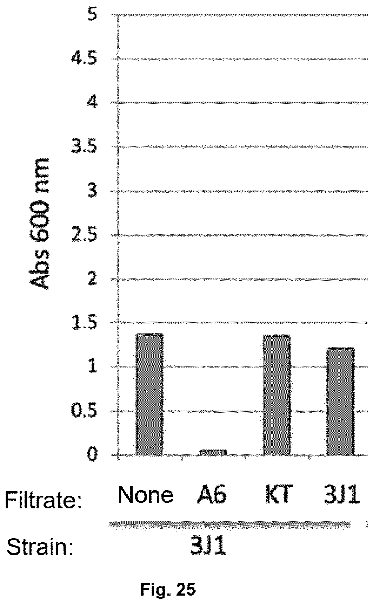

Subsequently, the absorbance of the different cultures was taken and it was observed that the tube with 3J1 inoculum and 1 ml of filtered A6 supernatant did not grow (Figure 25).

### Example 10. Effect on plant growth-promoting rhizobacteria

Assays were carried out to evaluate the effect of different *Bacillus* strains on the microbiota of the soil in which iceberg lettuces are planted (in commercial plant trays). Plants divided into different groups were inoculated with the live strains and the autoclaved inactive strains (named by adding the letter X). For inoculations, the strains *Bacillus velezensis* A6, *Bacillus velezensis* FZB42, *Bacillus amyloliquefaciens* subsp. *amyloliquefaciens Fukumoto* DSM7, B. *velezensis* XT1 and *Bacillus amyloliquefaciens* D747.

The inoculations were carried out, on three occasions, with an interval of 22 days between the first and second inoculation, and 12 days between the second and third inoculation, this third inoculation being in the field. The plants were collected 74 days after the first inoculation. They were kept under controlled conditions of eight hours of light, 26 °C temperature and irrigation with 3 ml of water for each plant, every 3 days, until they were transplanted in the field, on day 24 of the first inoculation.

An extraction of nucleic acids was carried out from a soil sample (specifically 0.5 g of soil from the rhizosphere of each plant, by means of FastPrep technology, with which molecular characterisation work was carried out), library preparation and Illumina sequencing were carried out at the IPBLN Genomics Center (CSIC, Granada, Spain).

For the sequencing analysis process, Mothur v1.39 software was used for the analysis of the raw sequencing data. Paired-end reads were merged into contigs that underwent quality trimming to avoid the generation of ambiguous bases in the overlap region arising from differences in the overlap. All contigs with homopolymers longer than 8 bp and showing ambiguous bases were removed. The Needleman criterion was used for the remaining contigs after aligning with the full SiLVA SEED v128 database, calculated using the k-nearest neighbour method with a k-mer size of 8. All sequences that failed in forward and reverse primer alignment were removed. Subsequently, chimeric sequences were identified using the VSEARCH algorithm implemented in mothur. Operational taxonomic units (OTUs) were constructed using non-chimeric sequences after taxonomic classification in the MiDAS database and used to construct via the abundance-based greedy clustering algorithm using a 97% cutoff for Prokarya. As a last resort, single OTUs were considered failures and removed.

In the case of the rhizosphere of plants inoculated with A6, as well as that of DSM7, D747 and KT, it is observed at what family level Gemmatinomonadaceae appears, which is not present in the sample without addition of bacteria (M9), or in the sample FZB42. Moreover, the appearance of the families Rubrobacteriadaceae and Nitrosomonadaceae and Sphingomonadaceae can be observed, and more significantly from the Bacillaceae family that were not present in the control sample without bacteria (M9), but that appear with the addition of all the microorganisms. Moreover, a reduction of the Tepidisphaeraceae family is observed as well as the Geodermatophilaceae family and the disappearance of the Nocardiodaceae family (the latter is present when samples D747 and KT were added).

In the case of the Nitrosomonadaceae family, species of this same family, it has been associated with nitrification via comamox of agricultural soils fertilised with nitrogen fertilizers (Lee, C., Hu, H. W., Chen, Q. L., Chen, D., & He, J. Z. (2019). Soil Biology and Biochemistry, 138, 107609.). In the case of the Sphingomonadaceae family, it is made up of four genera, which are *Sphingomonas, Sphingobium, Novosphingobium,* and *Sphingopyxis,* the genus *Sphingomonas appearing* when the samples are analysed at the genus level, describing various species of this genus associated with the promotion of plant growth, even promoting the appearance of other species during water stress (Luo, Y., et al. (2019). Frontiers in Microbiology, 10, 1221). These species of the genus *Sphingomonas* that promote plant growth include *Sphingomonas panaciterrae* (Sultana, R., et al, (2024). Heliyon, 10(3)), or *Sphinogomonas sediminicola* (Mazoyon, C., et al, (2023). Microorganisms, 11(8), 2061). Of course, the appearance of species of the Bacillaceae family (especially the genus *Bacillus)* are associated in many cases with the promotion of plant growth. The disappearance of the Nocardiaceae family is equally of note, within which numerous plant pathogens have been described. Therefore, the addition of the bacteria of the invention promotes the improvement of the soil microbiota.

## Claims

1. A *Bacillus velezensis* strain with deposit number CECT 30599 or a mutant thereof.

2. The mutant according to claim 1, wherein said mutant is obtained from the *Bacillus velezensis* strain with the deposit number CECT 30599 and wherein said mutant maintains the following capabilities:
- improve the resistance of a plant to drought,
- promote germination in a plant,
- promote the growth of a plant,
- control of pathogenic microorganisms in a plant,
- reduce the water supply to a plant,
- promote flowering in a plant, and/or
- increase the proportion of plant growth-promoting rhizobacteria in the soil where a plant is grown.

3. A cell culture of the *B. velezensis* strain CECT 30599 according to claim 1 or a mutant thereof according to claim 1 or 2.

4. An inactivated cell of the *B. velezensis* strain CECT 30599 according to claim 1 or the mutant according to claim 1 or 2.

5. A spore of the *B. velezensis* strain CECT 30599 according to claim 1 or the mutant according to claim 1 or 2.

6. A composition comprising one or more agriculturally acceptable compounds and:
i) the *B. velezensis* strain according to claim 1,
ii) the mutant according to claim 1 or 2,
iii) the cell culture according to claim 3,
iv) the inactivated cell according to claim 4, or
v) the spore according to claim 5.

7. A method for:
- improving the resistance of a plant to drought,
- promoting the germination in a plant,
- promoting the growth of a plant,
- the control of pathogenic microorganisms in a plant,
- reducing the water supply to a plant,
- promoting flowering in a plant, and/or
- increasing the proportion of plant growth-promoting rhizobacteria in the soil where a plant is cultivated,
wherein said method comprises applying to the plant, to a part thereof or the surroundings thereof, an effective amount of:
i) the *B. velezensis* strain according to claim 1,
ii) the mutant according to claim 1 or 2,
iii) the cell culture according to claim 3,
iv) the inactivated cell according to claim 4,
v) the spore according to claim 5, or
vi) the composition according to claim 6.

8. The method according to claim 7, wherein the strain i), the mutant ii), the culture iii), the inactivated cell iv), the spore v) or the composition vi) is applied to:
- the roots, leaves, fruits, flowers, calluses, tubers, stems or seeds of the plant or any combination thereof, and/or
- earth, soil, compost, mulch, leaf litter, sawdust, straw, pine straw, wood chips, gravel, plant growing medium, other material in a bed surrounding the plant, or any combination thereof.

9. The method according to claim 7 or 8, wherein the plant is a crop plant, preferably selected from the list consisting of a horticultural plant, woody plant, extensive crop plant, fruit plant, forage plant and oil plant.

10. A use of:
i) the *B. velezensis* strain according to claim 1,
ii) the mutant according to claim 1 or 2,
iii) the cell culture according to claim 3,
iv) the inactivated cell according to claim 4,
v) the spore according to claim 5, or
vi) the composition according to claim 6,
for
- improving the resistance of a plant to drought,
- promoting the germination in a plant,
- promoting the growth of a plant,
- the control of pathogenic microorganisms in a plant,
- reducing the water supply to a plant,
- promoting flowering of a plant, and/or
- increasing the proportion of plant growth-promoting rhizobacteria in the soil where a plant is grown.

11. The use according to claim 10 wherein the plant is a crop plant, preferably selected from the list consisting of a horticultural plant, woody plant, extensive crop plant, fruit plant, forage plant and oil plant.

12. A plant or part of a plant comprising:
i) the *B. velezensis* strain according to claim 1,
ii) the mutant according to claim 1 or 2,
iii) the cell culture according to claim 3, or
iv) the inactivated cell according to claim 4,
v) the spore according to claim 5, or
vi) the composition according to any one of claims 4 to 6.

13. The part of a plant according to claim 12, wherein said part is selected from the list consisting of root, leaf, fruit, flower, callus, tuber, stem and seed of the plant.

14. The plant according to claim 12 or part of a plant according to claim 12 or 13, wherein the plant wherein the plant is a crop plant, preferably selected from the list consisting of a horticultural plant, woody plant, extensive crop plant, fruit plant, forage plant and oil plant.

15. A method for obtaining a cell culture, wherein said method comprises:
(a) inoculating the B. *velezensis* strain according to claim 1, the mutant according to claim 1 or 2, or the spore according to claim 4 in a culture medium, and
(b) subjecting the inoculated culture medium from step (a) to conditions suitable for the growth of the strain or mutant.
